(19) Europäisches Patentamt
European Patent Office
Office européen des brevets

(11) Publication number: **0 463 456 A1**

(12) **EUROPEAN PATENT APPLICATION**

(21) Application number: **91109547.9**

(22) Date of filing: **11.06.91**

(51) Int. Cl.[5]: **C07F 9/572**, A61K 31/675, C07D 207/32

(30) Priority: **24.06.90 US 544701**

(43) Date of publication of application: **02.01.92 Bulletin 92/01**

(84) Designated Contracting States: **DE FR GB IT**

(71) Applicant: **E.R. SOUIBB & SONS, INC.**
**P.O.Box 4000**
**Princeton New Jersey 08543-4000(US)**

(72) Inventor: **Karanewsky, Donald S.**
**32 Ellsworth Drive**
**Robbinsville, NJ(US)**

(74) Representative: **Vossius & Partner**
**Siebertstrasse 4 P.O. Box 86 07 67**
**W-8000 München 80(DE)**

(54) **Phosphorus-containing HMG-CoA reductase inhibitors, new intermediates and their use.**

(57) Compounds which are useful as inhibitors of cholesterol biosynthesis and thus as hypocholesterolemic agents are provided which have the structure

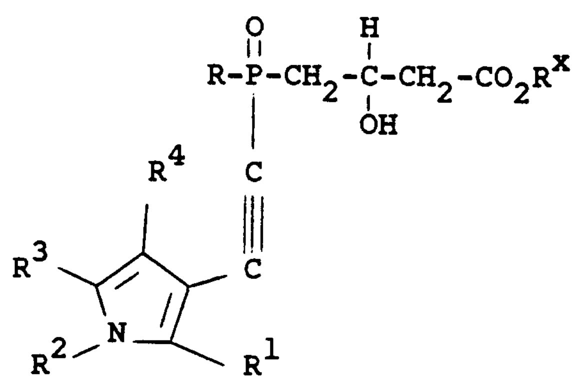

wherein R is OH, or salts thereof or lower alkoxy;
$R^x$ is H or alkyl;
$R^1$ is lower alkyl;
$R^2$ is lower alkyl;
$R^3$ is phenyl or substituted phenyl; and
$R^4$ is phenyl or substituted phenyl.

New pharmaceutical compositions containing such compounds especially oral dosage forms, and use of such compounds for the preparation of a pharmaceutical composition to inhibit cholesterol biosynthesis are also described.

EP 0 463 456 A1

The present invention relates to new orally active phosphorus-containing compounds which inhibit the activity of 3-hydroxy-3-methylglutaryl-coenzyme A reductase and thus are useful in inhibiting cholesterol biosynthesis, to hypocholesterolemic compositions containing such compounds, including oral dosage forms thereof, to a process for preparing such compounds, to new intermediates formed in the preparation of such compounds, and to the use of such compounds for the preparation of a pharmaceutical composition for such purposes.

F. M. Singer et al., Proc. Soc. Exper. Biol. Med., 102, 370 (1959) and F. H. Hulcher, Arch. Biochem. Biophys., 146, 422 (1971) disclose that certain mevalonate derivatives inhibit the biosynthesis of cholesterol.

Endo et al in U. S. Patents Nos. 4,049,495, 4,137,322 and 3,983,140 disclose a fermentation product which is active in the inhibition of cholesterol biosynthesis. This product is called compactin and was reported by Brown et al., (J. Chem. Soc. Perkin I. 1165 (1976)) to have a complex mevalonolactone structure.

GB 1,586,152 discloses a group of synthetic compounds of the formula

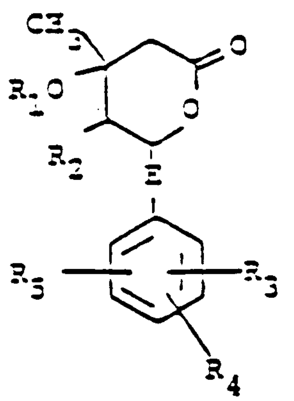

in which E represents a direct bond, a $C_{1-3}$ alkylene bridge or a vinylene bridge and the various R's represent a variety of substituents.

The activity reported in the U.K. patent is less than 1% that of compactin.

U. S. Patent No. 4,375,475 to Willard et al discloses hypocholesterolemic and hypolipemic compounds having the structure

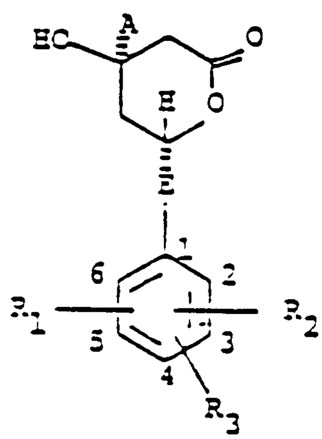

wherein A is H or methyl; E is a direct bond, $-CH_2-$, $-CH_2-CH_2-$, $-CH_2-CH_2-CH_2-$ or $-CH=CH-$; $R_1$, $R_2$ and $R_3$ are each selected from H, halogen, $C_{1-4}$ alkyl, $C_{1-4}$ haloalkyl, phenyl, phenyl substituted by halogen, $C_{1-4}$ alkoxy, $C_{2-8}$ alkanoyloxy, $C_{1-4}$ alkyl, or $C_{1-4}$ haloalkyl, and $OR_4$ in which $R_4$ is H, $C_{2-8}$ alkanoyl, benzoyl, phenyl, halophenyl, phenyl $C_{1-3}$ alkyl, $C_{1-9}$ alkyl, cinnamyl, $C_{1-4}$ haloalkyl, allyl, cycloalkyl-$C_{1-3}$-alkyl, adamantyl-$C_{1-3}$-alkyl, or substituted phenyl $C_{1-3}$-alkyl in each of which the substituents are selected from halogen, $C_{1-4}$ alkoxy, $C_{1-4}$ alkyl, or $c_{1-4}$ haloalkyl; and the corresponding dihydroxy acids resulting from the hydrolytic opening of the lactone ring, and the pharmaceutically acceptable salts of said acids, and the $C_{1-3}$ alkyl and phenyl, dimethylamino or acetylamino substituted $C_{1-3}$-alkyl esters of the dihydroxy acids; all of the compounds being the enantiomers having a 4 R configuration in the tetrahydropyran moiety

of the trans racemate shown in the above formula.

WO 84/02131 (PCT/EP83/00308) (based on U. S. application Serial No. 443,668, filed November 22, 1982, and U. S. application Serial No. 548,850, filed November 4, 1983), filed in the name of Sandoz AG discloses heterocyclic analogs of mevalono lactone and derivatives thereof having the structure

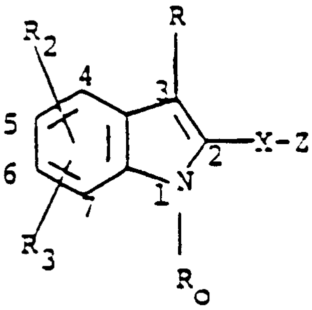

wherein one of R and $R_o$ is

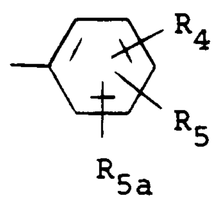

and the other is primary or secondary $C_{1-6}$ alkyl, $C_{3-6}$ cycloalkyl or phenyl-$(CH_2)_m$-,

wherein $R_4$ is hydrogen, $C_{1-4}$ alkyl, $C_{1-4}$ alkoxy, (except t-butoxy), trifluoromethyl, fluoro, chloro, phenoxy or benzyloxy,

$R_5$ is hydrogen, $C_{1-3}$ alkyl, $C_{1-3}$ alkoxy, trifluoromethyl, fluoro, chloro, phenoxy or benzyloxy,

$R_{5a}$ is hydrogen, $C_{1-2}$ alkyl, $C_{1-2}$ alkoxy, fluoro or chloro, and

m is 1, 2 or 3,

with the provisos that both $R_5$ and $R_{5a}$ must be hydrogen when $R_4$ is hydrogen, $R_{5a}$ must be hydrogen when $R_5$ is hydrogen, not more than one of $R_4$ and $R_5$ is trifluoromethyl, not more than one of $R_4$ and $R_5$ is phenoxy and not more than one of $R_4$ and $R_5$ is benzyloxy,

$R_2$ is hydrogen, $C_{1-4}$ alkyl, $C_{3-6}$ cycloalkyl, $C_{1-4}$ alkoxy (except t-butoxy), trifluoromethyl, fluoro, chloro, phenoxy or benzyloxy,

$R_3$ is hydrogen, $C_{1-3}$ alkyl, $C_{1-3}$ alkoxy, trifluoromethyl, fluoro, chloro, phenoxy or benzyloxy, with the provisos that $R_3$ must be hydrogen when $R_2$ is hydrogen, not more than one of $R_2$ and $R_3$ is trifluoromethyl, not more than one of $R_2$ and $R_3$ is phenoxy, and not more than one of $R_2$ and $R_3$ is benzyloxy.

X is $-(CH_2)_n-$ or $-CH=CH-$ (n = 0, 1, 2 or 3),

Z is

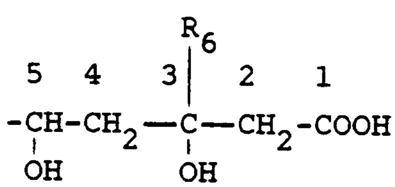

II

wherein $R_6$ is hydrogen or $C_{1-3}$ alkyl in free acid form or in the form of a physiologically-hydrolysable and -acceptable ester or a δ lactone thereof or in salt form.

GB 2162-179-A discloses naphthyl analogues of mevalolactone useful as cholesterol biosynthesis inhibitors having the structure

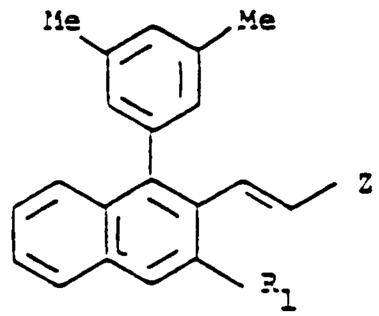

wherein $R_1$ = 1-3C alkyl;
Z is a gp. of formula $Z_1$ or $Z_2$:

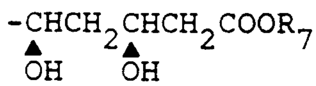

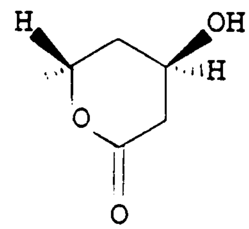

($Z_1$) ($Z_2$)

$R_7$ = H, a hydrolysable ester gp. or a cation.

European Patent No. 164-698-A discloses preparation of lactones useful as anti-hypercholesterolemic agents by treating an amide with an organic sulphonyl halide $R^5SO_2X$, then removing the protecting group Pr.

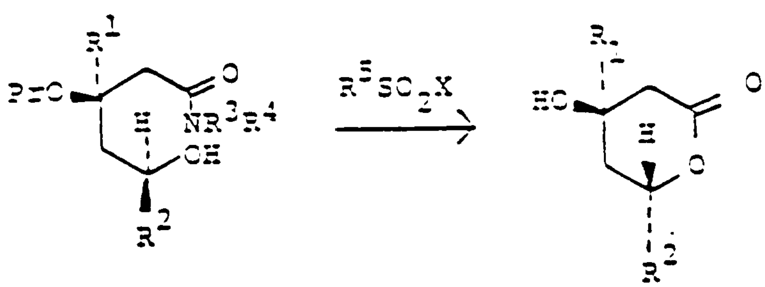


wherein X = halo;
Pr = a carbinol-protecting group;
$R^1$ = H or $CH_3$;
$R^3$, $R^4$ = H, 1-3C alkyl or phenyl-(1-3C alkyl), the phenyl being optionally substituted by 1-3C alkyl, 1-3C alkoxy or halo;
$R^2$ = a group of formula (A) or (B):

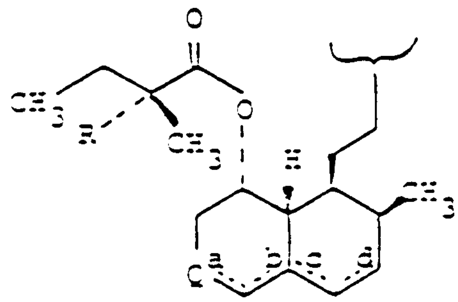

(A)

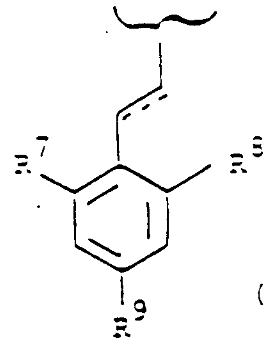

(B)

Q =

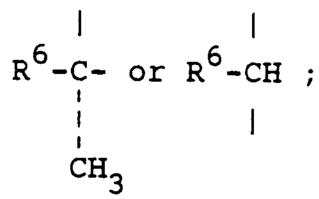

$R^6$ = H or OH;
R = H or $CH_3$;
a, b, c and d = optional double bonds;
$R^7$ = phenyl or benzyloxy, the ring in each case being optionally substituted by 1-3C alkyl or halo;
$R^8$, $R^9$ = 1-3C alkyl or halo;
$R^5$ = 1-3C alkyl, phenyl or mono- or di-(1-3C alkyl)phenyl.

Anderson, Paul Leroy, Ger. Offen. DE 3,525,256 discloses naphthyl analogs of mevalonolactones of the structure

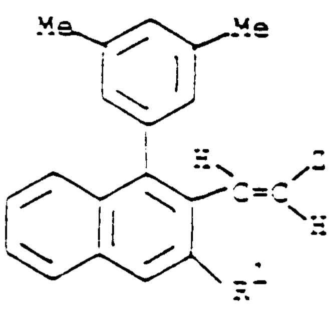

I

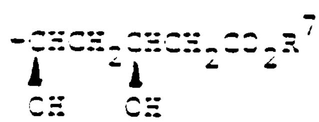

Q

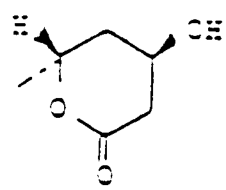

Q'

wherein $R^1$ is alkyl, Z = Q, $Q^1$; $R^7$ = H, or a hydrolyzable ester group useful as inhibitors of cholesterol biosynthesis and in treatment of atherosclerosis.

WO 8402-903 (based on U.S. application Serial No. 460,600, filed January 24, 1983) filed in the name of Sandoz AG discloses mevalono-lactone analogues useful as hypolipoproteinaemic agents having the structure

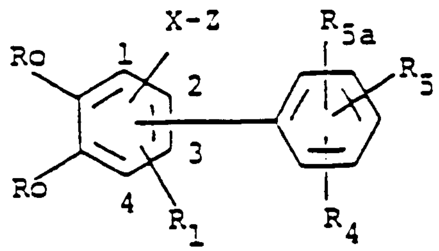

I

wherein the two groups Ro together form a radical of formula

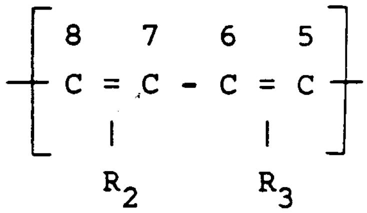

or $-(CH_2)_4-$

wherein $R_2$ is hydrogen, $C_{1-4}$ alkyl, $C_{1-4}$ alkoxy, (except t-butoxy), trifluoromethyl, fluoro, chloro, phenoxy or benzyloxy,

$R_3$ is hydrogen, $C_{1-3}$ alkyl, $C_{1-3}$ alkoxy, trifluoromethyl, fluoro, chloro, phenoxy or benzyloxy, with the provisos that not more than one of $R_2$ and $R_3$ is trifluoromethyl, not more than one of $R_2$ and $R_3$ is phenoxy, and not more than one of $R_2$ and $R_3$ is benzyloxy,

$R_1$ is hydrogen, $C_{1-6}$ alkyl, fluoro, chloro or benzyloxy,

$R_4$ is hydrogen, $C_{1-4}$ alkyl, $C_{1-4}$ alkoxy, (except t-butoxy), trifluoromethyl, fluoro, chloro, phenoxy or benzyloxy,

$R_5$ is hydrogen, $C_{1-3}$ alkyl, $C_{1-3}$ alkoxy, trifluoromethyl, fluoro, chloro, phenoxy or benzyloxy,

$R_{5a}$ is hydrogen, $C_{1-2}$ alkyl, $C_{1-2}$ alkoxy, fluoro or chloro, and with the provisos that not more than one of $R_4$ and $R_5$ is trifluoromethyl, not more than one of $R_4$ and $R_5$ is phenoxy and not more than one of $R_4$ and $R_5$ is benzyloxy,

X is $-(CH_2)_n-$,

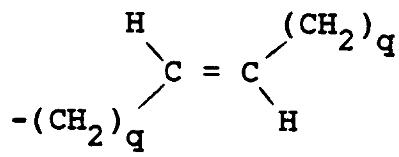

wherein n is 0, 1, 2 or 3 and both q's are 0 or one is 0 and the other is 1,

Z is

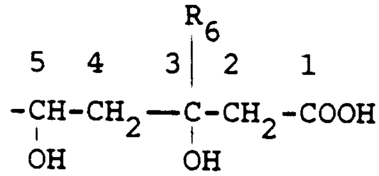

II

wherein $R_6$ is hydrogen or $C_{1-3}$ alkyl, with the general proviso that -X-Z and the $R_4$ bearing phenyl group are ortho to each other;

in free acid form or in the form of a physiologically-hydrolysable and acceptable ester or a $\delta$ lactone thereof or in salt form.

U. S. Patent No. 4,613,610 to Wareing (assigned to Sandoz) discloses a series of 7-pyrazolo-3,5-dihydrohept-6-enoic acid HMG-CoA reductase inhibitors of the structure

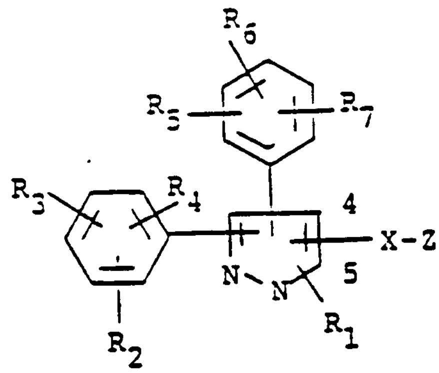

wherein

$R_1$ is $C_{1-6}$alkyl not containing an asymmetric carbon atom,

each of $R_2$ and $R_5$ is independently hydrogen, $C_{1-3}$alkyl, n-butyl, i-butyl, t-butyl, $C_{1-3}$alkoxy,n-butoxy, i-butoxy, trifluoromethyl, fluoro, chloro, phenyl, phenoxy or benzyloxy,

each of $R_3$ and $R_6$ is independently hydrogen, $C_{1-3}$alkyl, $C_{1-3}$alkoxy, trifluoromethyl, fluoro, chloro, phenoxy or benzyloxy,

each of $R_4$ and $R_7$ is independently hydrogen, $C_{1-2}$alkyl, $C_{1-2}$alkoxy, fluoro or chloro, with the provisos that not more than one of $R_2$ and $R_3$ is trifluoromethyl, not more than one of $R_2$ and $R_3$ is phenoxy, not more than one of $R_2$ and $R_3$ is benzyloxy, not more than one of $R_5$ and $R_6$ is trifluoromethyl, not more than one of $R_5$ and $R_6$ is phenoxy, and not more than one of $R_5$ and $R_6$ is benzyloxy,

X is $-(CH_2)_m-$, -CH=CH-, $-CH=CH-CH_2-$ or $-CH_2-CH=CH-$, wherein m is 0, 1, 2 or 3, and

Z is

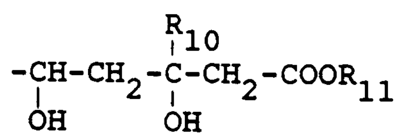

or

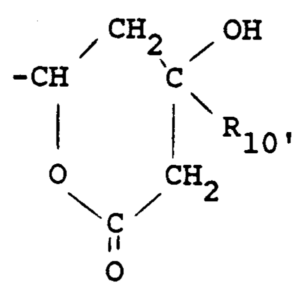

wherein $R_{10}$ is hydrogen or $C_{1-3}$-alkyl, and $R_{11}$ is hydrogen, $R_{12}$ or M, wherein

$R_{12}$ is a physiologically acceptable and hydrolyzable ester group, and

M is a cation,

with the provisos that (i) the -X-Z group is in the 4- or 5-position of the pyrazole ring, and (ii) the $R_1$ group and the -X-Z group are ortho to each other.

WO 8607-054A (Sandoz-Erfindungen) discloses imidazole analogues of mevalonolactone, useful for treating hyperlipoproteinaemia and atherosclerosis, which have the formula

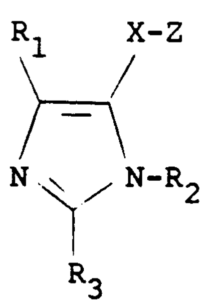

(I)

$R_1$ = alkyl, cycloalkyl, adamantyl-1 or $R_4$, $R_5$, $R_6$-substituted phenyl (gp. A);

$R_2$ = alkyl, cycloalkyl, adamantyl-1 or $R_7$, $R_8$, $R_9$-substituted phenyl (gp. B);
$R_3$ = H, alkyl, cycloalkyl, adamantyl-1, styryl or $R_{10}$, $R_{11}$, $R_{12}$-substituted phenyl (gp, C);
X = $-(CH_2)_m-$, -CH=CH-, -CH=CH-$CH_2$- or -$CH_2$-CH=CH-;
m = 0-3;
Z = -CH(OH)-$CH_2$-C($R_{13}$)(OH)-$CH_2$-$COOR_{14}$ (gp. a), -Q-$CH_2$-C($R_{13}$)(OH)-$CH_2$-$COOR_{14}$ (gp. c) or a gp. of formula (b):

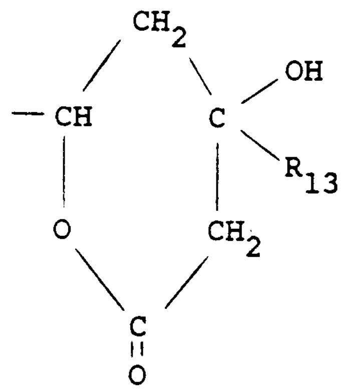

Q = CO or -C($OR_{15}$)$_2$-;
$R_{15}$ = primary or sec. alkyl; each $R_{15}$ being the same;
or $R_{15}$ + $R_{15}$ = $(CH_2)_2$ or $(CH_2)_3$;
$R_{13}$ = H or 1-3C alkyl;
$R_{14}$ = H, $R_{16}$ or M;
$R_{16}$ = ester gp.;
M = cation;
provided that Z may be gp. (c) only when X is CH=CH or $CH_2$-CH=CH and/or when $R_{13}$ = 1-3C alkyl;
$R_4$, $R_7$ and $R_{10}$ = 1-3C alkyl, n-, i- or t-butyl, 1-3C alkoxy, n- or i-butoxy, $CF_3$, F, Cl, Br, phenyl, phenoxy or benzyloxy;
$R_5$, $R_8$ and $R_{11}$ = H, 1-3C alkyl, 1-3C alkoxy, $CF_3$, F, Cl, Br, $COOR_{17}$, N($R_{19}$)$_2$, phenoxy or benzyloxy;
$R_{17}$ = H, $R_{18}$ or M;
$R_{18}$ = 1-3C alkyl, n, i- or t-butyl or benzyl;
$R_{19}$ = alkyl;
$R_6$, $R_9$ and $R_{12}$ = H, 1-2C alkyl, 1-2C alkoxy, F or Cl; provided that
(1) not more than one substituent of each of gps. A, B and C is $CF_3$, not more than one substituent of each of gps. A, B and C is phenoxy, and not more than one substituent of each of gps, A, B and C is benzyloxy;
(2) when Z is gp. (c; Q = C($OR_{15}$)$_2$), the compound is in free base form and either (i) $R_{14}$ is $R_{16}$ and each $R_{17}$ is independently $R_{18}$ or (ii) $R_{14}$ is M and each $R_{17}$ is independently $R_{18}$ or M; and
(3) when $R_{14}$ and/or at least one $R_{17}$ is M, the compound is in free base form.

Unless otherwise stated, all "alkyl" gps. are 1-6C and do not contain an asymmetric C; and "cycloalkyl" has 3-7C.

WO 8603-488-A (Sandoz AG) discloses indene analogues of mevalolactone, useful as hypolipoproteinaemia and anti-atherosclerotic agents, in free acid form or in the form of an ester or delta-lactone or in salt form which have the formula

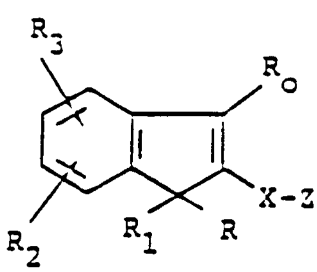

(I)

R = H or primary or secondary 1-6C alkyl;

$R_1$ = primary or secondary 1-6C alkyl;

or R + $R_1$ = $(CH_2)_m$ or (Z)-$CH_2$-CH = CH-$CH_2$;

m = 2-6;

m = 2-6

$R_o$ = 1-6C alkyl, 3-7C cycloalkyl or $R_4$, $R_5$, $R_6$-substituted phenyl;

$R_2$, $R_4$ = H, 1-4C alkyl, 1-4C alkoxy (except t-butoxy), $CF_3$, F, Cl, phenoxy or benzyloxy;

$R_3$ and $R_5$ = H, 1-3C alkyl, 1-3C alkoxy, $CF_3$, F, Cl, phenoxy or benzyloxy;

$R_6$ = H, 1-2C alkyl, 1-2C alkoxy, F or Cl;

provided that there may only be one each of $CF_3$, phenoxy or benzyloxy on each of the phenyl and indene rings;

X = $(CH_2)_n$ or -$(CH_2)_q$-CH = $CH(CH_2)_q$-;

n = 1-3;

both q's = 0, or one is 0 and the other is 1;

Z = -Q-$CH_2$-C($R_{10}$)(OH)-$CH_2$COOH, in free acid form or in the form of an ester or delta-lactone or salt;

Q = CO, -C($OR_7$)$_2$- or CHOH;

$R'_{7s}$ = the same primary or secondary 1-6C alkyl, or together are $(CH_2)_2$ or $(CH_2)_3$;

$R_{10}$ = H or 1-3C alkyl;

provided that Q may be other than CHOH only when X is CH = CH or $CH_2$-CH = CH and/or $R_{10}$ is 1-3C alkyl.

U. S. Patent No. 4,647,576 to Hoefle et al (Warner Lambert) discloses new C- and N-substituted pyrrole(s), useful as hypolipidaemic and hypocholesterolaemic agents, which have the formula

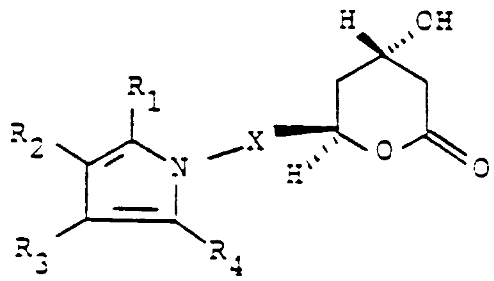

(I)

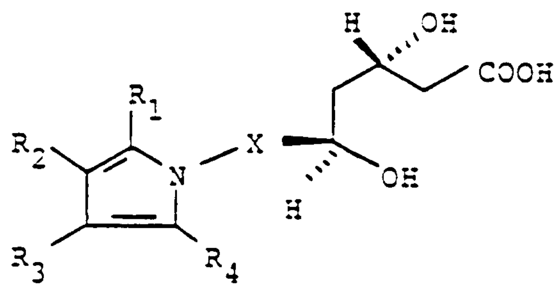

(II)

X = -$CH_2$-, -$CH_2CH_2$- or -$CH(CH_3)CH_2$-;

$R_1$ = 1- or 2-naphthyl; cyclohexyl; norbornenyl; phenyl optionally substituted by F, Cl, OH, $CF_3$, 1-4C alkyl, 1-4C alkoxy or 2-8C alkanoyloxy; 2-, 3- or 4-pyridinyl or their N-oxides; or

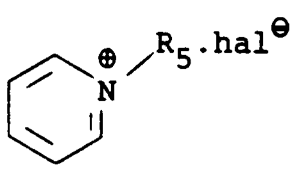

$R_5$ = 1-4C alkyl;

hal = chloride, bromide or iodide;

$R_2$ and $R_3$ = H, Cl, Br, CN, $CF_3$, phenyl, 1-4C alkyl, 2-8C carboalkoxy, -$CH_2OR_6$ or -$CH_2OCONHR_7$;

$R_6$ = H or 1-6C alkanoyl;
$R_7$ = alkyl or phenyl optionally substituted by Cl, Br or 1-4C alkyl;
or $R_2$ and $R_3$ together = $-(CH_2)_n-$, $-CH_2OCH_2-$, $-CON(R_8)CO-$ or $-CON(R_9)N(R_{10})CO-$;
n = 3 or 4;
$R_8$ = H, 1-6C alkyl, phenyl or benzyl;
$R_9$ and $R_{10}$ = H, 1-4C alkyl or benzyl;
$R_4$ = 1-4C alkyl, cyclopropyl, cyclobutyl or $CF_3$.

European patent application 0 221 025 A1 (Sandoz AG) discloses heterocyclic analogs of mevalonolactone and derivatives thereof having the formula

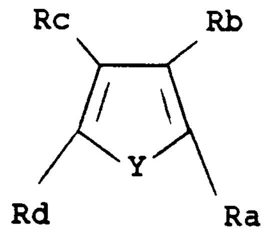

wherein

Ra is a group -X-Z, Rb is $R_2$, Rc is $R_3$, Rd is $R_4$ and Y is a group

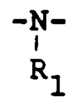

or

Ra is $R_1$, Rb is a group -X-Z, Rc is $R_2$, Rd is $R_3$ and Y is O, S or a group

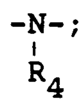

$R_1$, $R_2$, $R_3$ and $R_4$ independently are $C_{1-4}$ alkyl not containing an asymmetric carbon atom, $C_{3-7}$cycloalkyl or a ring

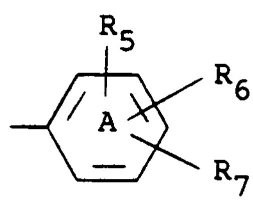

or in the case of $R_3$ and $R_4$ additionally hydrogen or for $R_3$ when Y is O or S

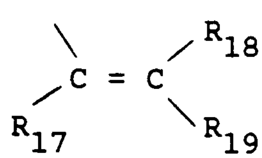

whereby $R_{17}$ is hydrogen or $C_{1-3}$alkyl and $R_{18}$ and $R_{19}$ are independently hydrogen, $C_{1-3}$alkyl or phenyl; each $R_5$ is independently hydrogen, $C_{1-3}$alkyl, n-butyl, i-butyl, t-butyl, $C_{1-3}$alkoxy,n-butoxy, i-butoxy, trifluoromethyl, fluoro, chloro, bromo, phenyl, phenoxy or benzyloxy; each $R_4$ is independently hydrogen,

$C_{1-3}$alkyl, $C_{1-3}$alkoxy, trifluoromethyl, fluoro, chloro, bromo, phenoxy or benzyloxy and each $R_7$ is independently hydrogen, $C_{1-2}$alkyl, $C_{1-2}$alkoxy, fluoro or chloro with the proviso that there may only be one each of trifluoromethyl, phenoxy or benzyloxy in each ring A present. X is $(CH_2)_m$ or $(CH_2)_qCH=CH-(CH_2)_q$, m is 0, 1, 2 or 3 and both q's are 0 or one is 0 and the other is 1.

Z is 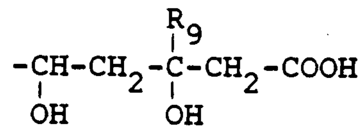

wherein $R_9$ is hydrogen or $C_{1-3}$alkyl, in free acid form or in the form of an ester of $\beta$-lactone thereof or in salt form as appropriate which compounds are indicated for use as hypolipoproteinemic and anti-atherosclerotic agents.

Tetrahedron Letters, 29, 929, 1988, discloses the synthesis of a 3-hydroxy-3-methyl-glutaryl coenzyme A reductase inhibitor of the structure

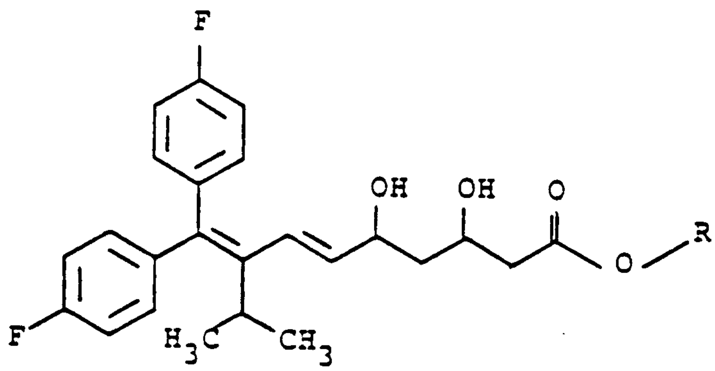

where R is Na or $C_2H_5$.

European patent application 127,848-A (Merck & Co, Inc.) discloses derivatives of 3-hydroxy-5-thia-$\omega$-aryl-alkanoic acids having the structural formula:

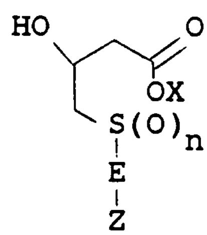

wherein Z is:

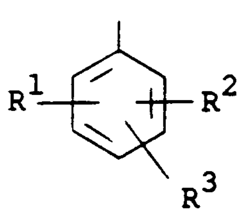

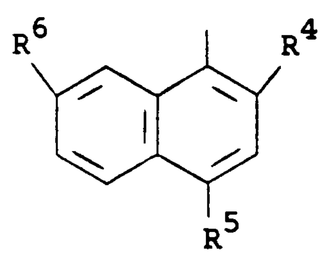

n is 0, 1 or 2;

E is $-CH_2-$, $-CH_2-CH_2-$, $-CH_2-CH_2-CH_2-$, $-CH=CH-CH_2-$; or $-CH_2-CH=CH-$;

$R_1$, $R_2$ and $R_3$ are, e.g., hydrogen, chloro, bromo, fluoro, $C_1$-alkyl, phenyl, substituted phenyl or $OR_7$ in

which $R_7$ is, e.g., hydrogen,

$C_{2-8}$alkanoyl, benzoyl, phenyl, substituted phenyl, $C_{1-9}$alkyl, cinnamyl, $C_{1-4}$haloalkyl, allyl, cycloalkyl-$C_{1-3}$alkyl, adamantyl-$C_{1-3}$-alkyl, or phenyl $C_{1-3}$ alkyl;

$R^4$, $R^5$ and $R^6$ are hydrogen, chloro, bromo, fluoro or $C_{1-3}$ alkyl; and

X is, e.g., hydrogen, $C_{1-3}$ alkyl, a cation derived from an alkali metal or is ammonium.

Those compounds have antihypercholesterolemic activity by virtue of their ability to inhibit 3-hydroxy-3-methylglutaryl-coenzyme A (HMG-CoA) reductase and antifungal activity.

French patent application 2,596,393 A filed on April 1, 1986 (Sanofi SA) discloses 3-carboxy-2-hydroxypropane-phosphonic acid derivatives including salts thereof which are useful as hypolipaemic agents and have the formula:

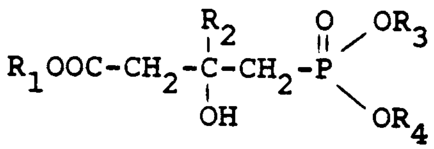

wherein $R_1$ and $R_2$ = H, lower alkyl or optionally substituted aralkyl;

$R_3$ and $R_4$ = H, lower alkyl or optionally substituted aryl or aralkyl.

These comounds are disclosed as giving greater reductions in cholesterol, triglyceride and phospholipid levels than meglutol.

European patent application 142,146-A (Merck & Co., Inc) discloses mevinolin-like compounds of the structural formula:

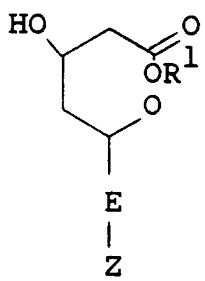

wherein:

$R^1$ is, e.g., hydrogen or $C_{1-4}$alkyl;

E is $-CH_2CH_2$, $-CH=CH-$, or $-(CH_2)_r-$; and

Z is

1)

wherein n is 0-2 and $R^{14}$ is halo or $C_{1-4}$alkyl; or

4)

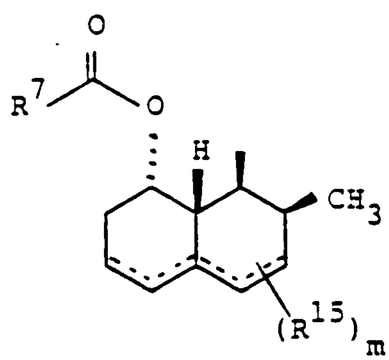

These compounds are HMG-CoA reductase inhibitors.

British Patent 2205838 (which corresponds to U.S. applications Serial No. 109,681, filed October 19,

1987, and Serial No. 053,238, filed May 22, 1987, parents of the subject application) discloses HMG CoA reductase inhibitors of the structure

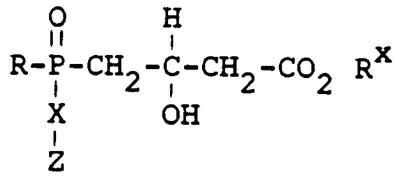

wherein R is OH or lower alkoxy, $R^x$ is H or lower alkyl, X is $-CH_2-$, $-CH_2CH_2-$, $-CH_2CH_2CH_2-$, $-CH=CH-$, $-C\equiv C-$ or $-CH_2O-$ (where O is linked to Z); and Z is a hydropholic anchor which includes

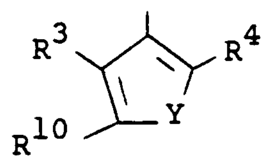

wherein one of $R^3$ and $R^4$ is

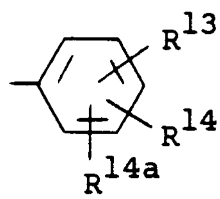

and the other is lower alkyl, cycloalkyl or phenyl-$(CH_2)_p$-, p is 0, 1, 2, 3 or 4;

$R^{10}$ is hydrogen, alkyl, cycloalkyl, adamantyl-1, or $-(CH_2)_q$

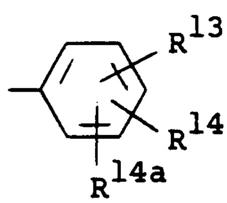

where $R^{13}$, $R^{14}$ and $R^{14a}$ are as defined above and q is 0, 1, 2, 3 or 4;

Y is 0, S or $NR^{10}$.

As will be seen, the compounds of the present invention represents a sub-genus and species of the invention disclosed in the latter British Patent.

In accordance with the present invention, there is provided orally active phosphorus-containing compounds which inhibit the enzyme 3-hydroxy-3-methylglutaryl-coenzyme A reductase (HMG-CoA Reductase) and thus are useful as orally active hypocholesterolemic agents and have the formula I

I

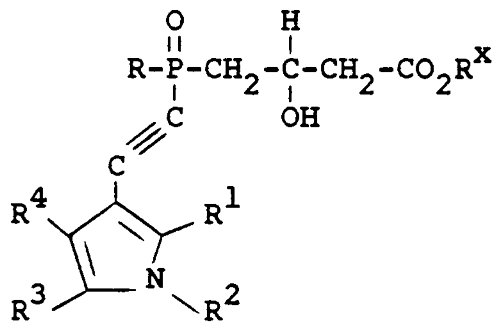

wherein R is OH or lower alkoxy;

$R^x$ is H or lower alkyl;

$R^1$ is lower alkyl;

$R^2$ is lower alkyl;

$R^3$ is phenyl or phenyl substituted with one, two or three lower alkyl, lower alkoxy (except t-butoxy) or halogen groups; and

$R^4$ is phenyl or phenyl substituted with one, two or three lower alkyl, lower alkoxy (except t-butoxy) or halogen groups;

and including pharmaceutically acceptable salts thereof.

In addition, in accordance with the present invention, a method is provided for preparing such formula I compounds which includes the steps of treating a cooled solution of an aldehyde compound of the structure II

II

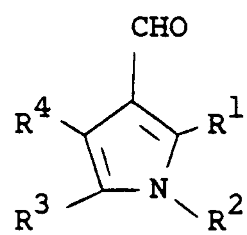

in dry inert organic solvent and dry chloroform with lithium bis(trimethylsilyl)amide under an inert atmosphere, treating the resulting reaction product with acetic anhydride and pyridine under an inert atmosphere, to form a trichloride compound of the structure III

III

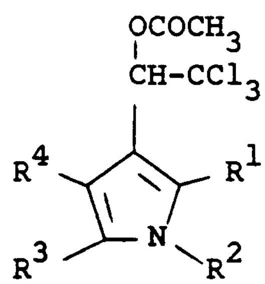

(a novel intermediate)

treating the trichloride with lead chloride or bromide and aluminum in dry inert organic solvent under an inert atmosphere to form a dichloride of the structure IV

IV

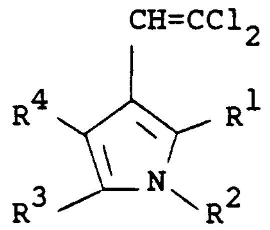

(a novel intermediate)

and employing the dichloride to form the compounds of formula I.

The terms "salt" and "salts" refer to basic salts formed with inorganic and organic bases. Such salts include ammonium salts, alkali metal salts like, lithium, sodium and potassium salts (which are preferred), alkaline earth metal salts like the calcium and magnesium salts, salts with organic bases, such as amine like salts, e.g., dicyclohexylamine salt, benzathine, N-methyl-D-glucamine, hydrabamine salts, salts with amino acids like arginine, lysine and the like. The nontoxic, pharmaceutically acceptable salts are preferred, although other salts are also useful, e.g., in isolating or purifying the product.

The term "lower alkyl" or "alkyl" as employed herein alone or as part of another group includes both straight and branched chain hydrocarbons, containing 1 to 12 carbons in the normal chain, preferably 1 to 7 carbons, such as methyl, ethyl, propyl, isopropyl, butyl, t-butyl, isobutyl, pentyl, hexyl, isohexyl, heptyl, 4,4-dimethylpentyl, octyl, 2,2,4-trimethylpentyl, nonyl, decyl, undecyl, dodecyl, the various branched chain isomers thereof, and the like as well as such groups including a halo-substituent, such as F, Br, Cl or I or $CF_3$, an alkoxy substituent, an aryl substituent, an alkyl-aryl substituent, a haloaryl substituent, a cycloalkyl substituent, an alkylcycloalkyl substituent, hydroxy, and alkylamino substituent, an alkanoylamino substituent, an arylcarbonylamino substituent, a nitro substituent, a cyano substituent, a thiol substituent or an alkylthio substituent.

The term "cycloalkyl" as employed herein alone or as part of another group includes saturated cyclic hydrocarbon groups containing 3 to 12 carbons, preferably 3 to 8 carbons, which include cyclopropyl, cyclobutyl, cyclopentyl, cyclohexyl, cycloheptyl, cyclooctyl, cyclodecyl and cyclododecyl, any of which groups may be substituted with 1 or 2 halogens, 1 or 2 lower alkyl groups, 1 or 2 lower alkoxy groups, 1 or 2 hydroxy groups, 1 or 2 alkylamino groups, 1 or 2 alkanoylamino groups, 1 or 2 arylcarbonylamino groups, 1 or 2 amino groups, 1 or 2 nitro groups, 1 or 2 cyano groups, 1 or 2 thiol groups, and/or 1 or 2 alkylthio groups.

The term "aryl" or "Ar" as employed herein refers to monocyclic or bicyclic aromatic groups containing from 6 to 10 carbons in the ring portion, such as phenyl, naphthyl, substituted phenyl or substituted naphthyl wherein the substituent on either the phenyl or naphthyl may be 1, 2 or 3 lower alkyl groups, halogens (Cl, Br or F), 1, 2 or 3 lower alkoxy groups, 1, 2 or 3 hydroxy groups, 1, 2 or 3 phenyl groups, 1, 2 or 3 alkanoyloxy group, 1, 2 or 3 benzoyloxy groups, 1, 2 or 3 haloalkyl groups, 1, 2 or 3 halophenyl groups, 1, 2 or 3 allyl groups, 1, 2 or 3 cycloalkylalkyl groups, 1, 2 or 3 adamantylalkyl groups, 1, 2 or 3 alkylamino groups, 1, 2 or 3 alkanoylamino groups, 1, 2 or 3 arylcarbonylamino groups, 1, 2 or 3 amino groups, 1, 2 or 3 nitro groups, 1, 2 or 3 cyano groups, 1, 2 or 3 thiol groups, and/or 1, 2 or 3 alkylthio groups with the aryl group preferably containing 3 substituents.

The term "aralkyl", "aryl-alkyl" or "aryl-lower alkyl" as used herein alone or as part of another group refers to lower alkyl groups as discussed above having an aryl substituent, such as benzyl.

The term "lower alkoxy", "alkoxy", or "aryloxy" or "aralkoxy" as employed herein alone or as part of another group includes any of the above lower alkyl, alkyl, aralkyl or aryl groups linked to an oxygen atom.

The term "lower alkylthio", "alkylthio", "arylthio" or "aralkylthio" as employed herein alone or as part of another group includes any of the above lower alkyl, alkyl, aralkyl or aryl groups linked to a sulfur atom.

The term "lower alkylamino", "alkylamino", "arylamino", "arylalkylamino" as employed herein alone or as part of another group includes any of the above lower alkyl, alkyl, aryl or arylalkyl groups linked to a nitrogen atom.

The term "alkanoyl" as used herein as part of another group refers to lower alkyl linked to a carbonyl group.

The term "halogen" or "halo" as used herein refers to chlorine, bromine, fluorine, iodine and $CF_3$, with chlorine or fluorine being preferred.

Preferred are those compounds of formula I wherein $R^1$ is isopropyl, $R^2$ is isopropyl, $R^3$ is phenyl, $R^4$ is substituted phenyl, such as halophenyl.

The compounds of formula I of the invention may be prepared according to the following reaction sequence and description thereof.

Reaction Sequence - Preparation of Compounds of Formula I

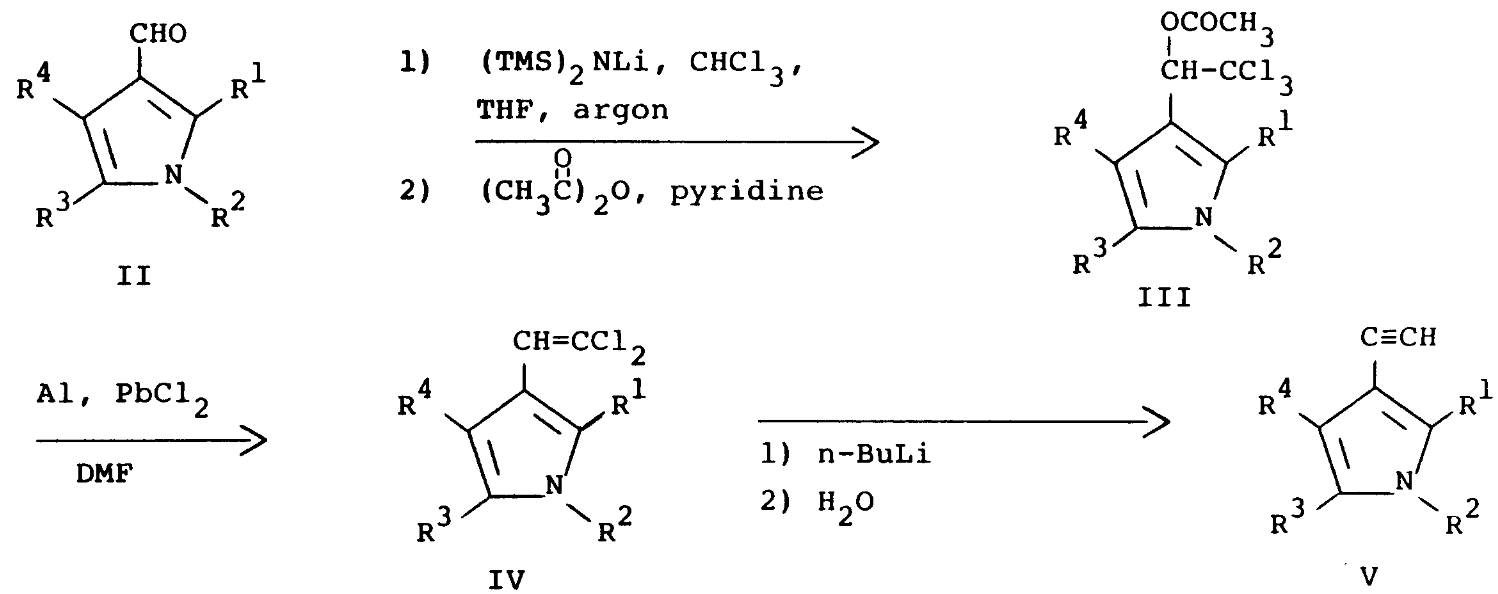


II III IV V

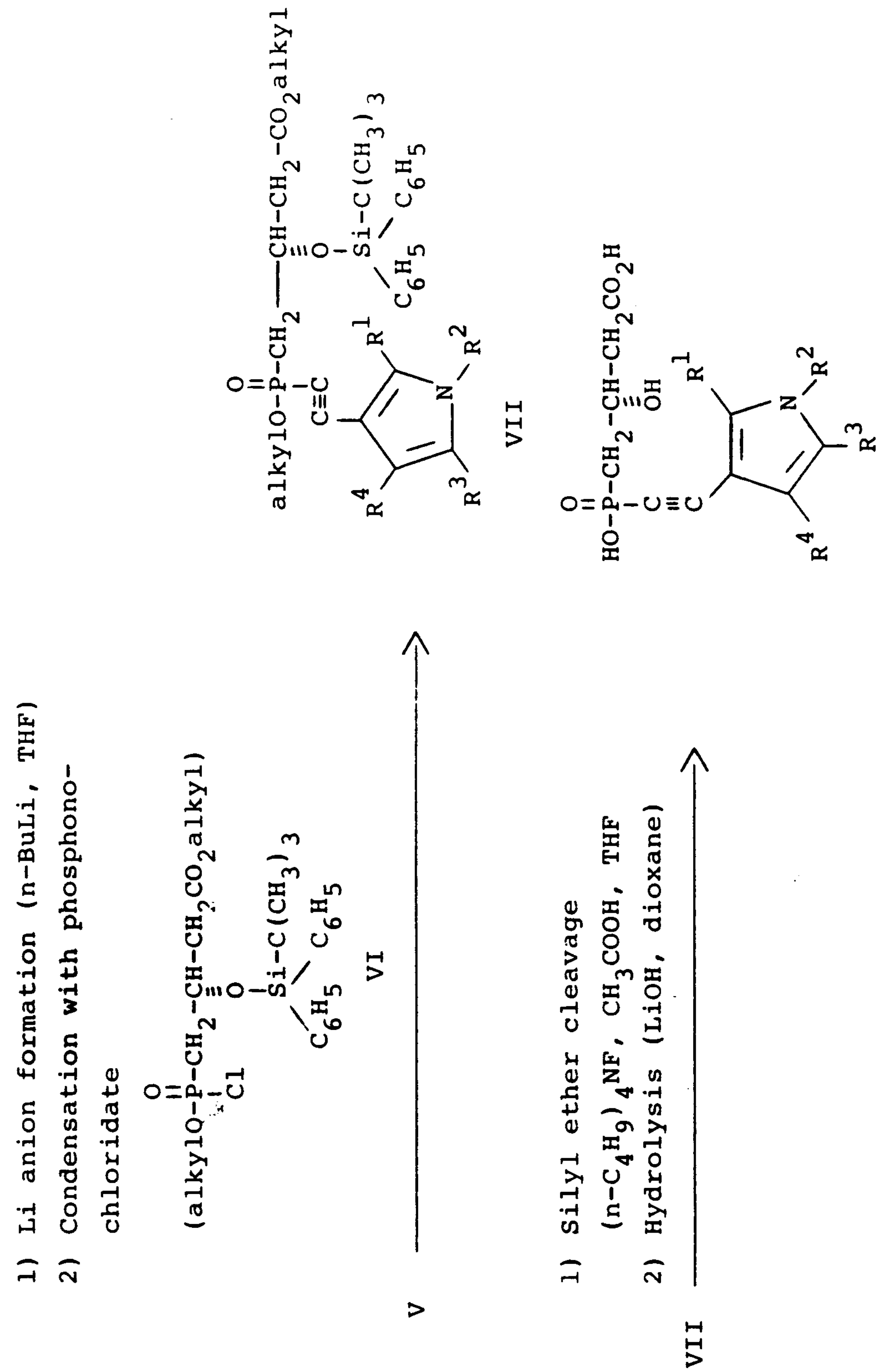


As seen in the above Reaction Sequence, compounds of Formula I may be prepared by treating aldehyde II in dry chloroform (molar ratio of II: chloroform of within the range of from about 1.1:1 to about 5:1) and dry inert organic solvent, such as tetrahydrofuran, with lithium bis(trimethylsilyl)amide (molar ratio of II:lithium compound of within the range of from about 1:1 to about 2:1), at a temperature within the range of from about -78° to about -40° C under an inert atmosphere such as argon; and then treating the resulting reaction product with acetic anhydride and pyridine (molar ratio of II:acetic anhydride of within the range of from about 1.5:1 to about 10:1), under an inert atmosphere, such as argon, to form trichloride III (a novel

intermediate).

Trichloride III is then treated with lead chloride (molar ratio of III:$PbCl_2$ of within the range of from about 1:0.05 to about 1:0.1) and aluminum foil (molar ratio of III:A1 of within the range of from about 1:1 to about 1:2), in the presence of an inert organic solvent such as dimethylformamide at a temperature within the range of from about 25° to about 75° C, to form dichloride IV (a novel intermediate). Compound IV is treated with a strong base such as n-butyllithium in an inert organic solvent such as tetrahydrofuran (THF) under an inert atmosphere (for example argon) to give V.

The phosphonochloridate VI (prepared as described in U.S. Patent No. 4,904,646), is dissolved in inert organic solvent such as THF, the solution is cooled to a temperature within the range of from about -90° C to about 0° C and preferably from about -85° C to about -30° C and treated with a cooled (same range as solution of phosphonochloridate VI) solution of the lithium anion of acetylene V employing a molar ratio of VI:V of within the range of from about 3:1 to about 1:1, and preferably from about 1.5:1 to about 2:1, to form the acetylenic phosphinate VII

VII

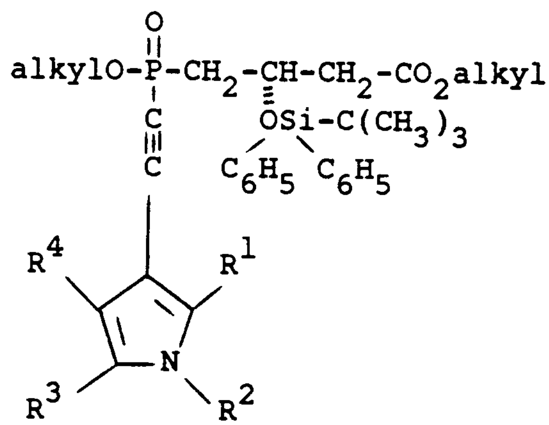

(a novel intermediate)

Acetylenic phosphinate VII may then be employed to prepare the various compounds of the present invention as follows.

Acetylenic phosphinate VII is converted to acetylenic phosphinate IA by subjecting VII to silyl ether cleavage by treating VII in an inert organic solvent such as tetrahydrofuran, with glacial acetic acid and tetrabutylammonium fluoride to form ester $IA^1$

$IA^1$

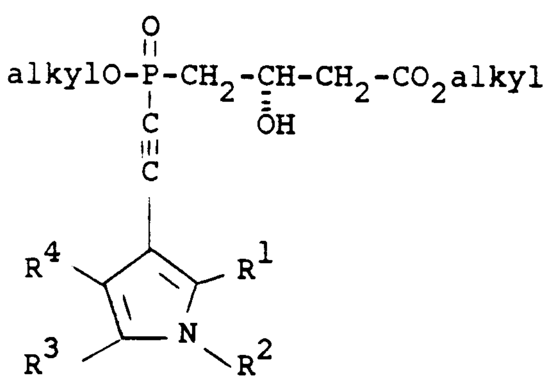

which may then be hydrolyzed to the corresponding basic salt or acid, that is, where $R^x$ is $R^{xa}$ which is ammonium, alkali metal, alkaline earth metal, an amine and the like, by treatment with strong base such as lithium hydroxide in the presence of dioxane, tetrahydrofuran or other inert organic solvent under an inert atmosphere such as argon, at 25° C, employing a molar ratio of base:ester $IA^1$ of within the range of from

about 1:1 to about 1.1:1 to form the corresponding basic salt $IA^2$

$IA^2$

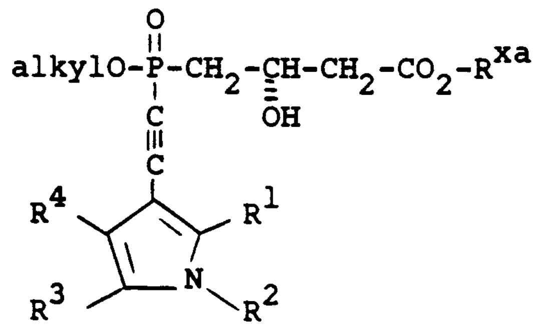

Compound $IA^2$ may then be treated with strong acid such as HCl to form the corresponding acid $IA^3$

$IA^3$

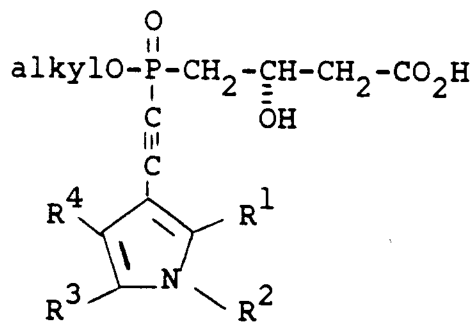

The ester $IA^1$ may be converted to the corresponding di-basic salt by treating ester $IA^1$ with strong base at 50-60°C employing a molar ratio of base:ester $IA^1$ of within the range of from about 2:1 to about 4:1 to form $IA^4$

$IA^4$

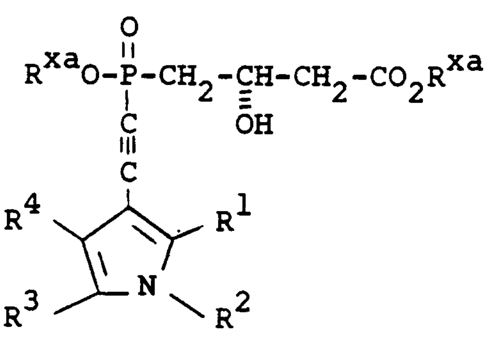

The dibasic salt $IA^4$ may be converted to the corresponding acid by treatment with strong acid such as HCl, to form acid IA.

The starting aldehyde compounds II are prepared following the procedure as set out in Example 1.

The various intermediates III, IV, V, and VII, also are part of the present invention. These novel intermediates may be represented by the following generic formulae:

X

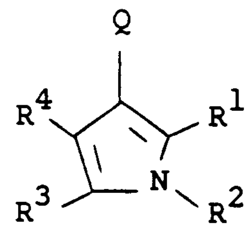

wherein Q is

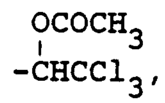

$-CH=CCl_2$, or $-C\equiv CH$; and

XI

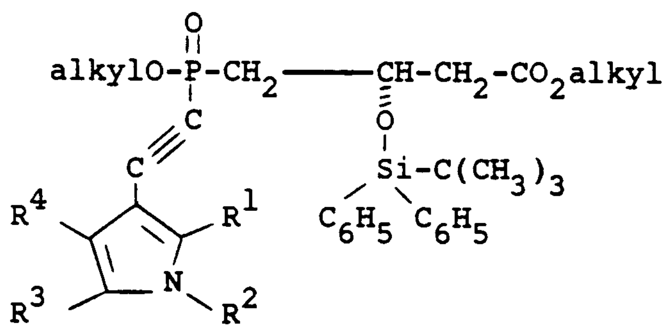

including all stereoisomers thereof, wherein $R^1$, $R^2$, $R^3$ and $R^4$ are as defined above.

The compounds of the invention may be prepared as racemic mixtures and may later be resolved to obtain the S-isomer which is preferred. However, the compounds of the invention may be prepared directly in the form of their S-isomers as described herein and in the working examples set out hereinafter.

The compounds of the invention are inhibitors of 3-hydroxy-3-methyl-glutaryl coenzyme A (HMG-CoA) reductase and thus are useful in inhibiting cholesterol biosynthesis as demonstrated by the following tests.

1) Rat Hepatic HMG-CoA Reductase

Rat hepatic HMG-CoA reductase activity is measured using a modification of the method described by Edwards (Edwards, P.A., et al., J. Lipid Res. 20:40, 1979). Rat hepatic microsomes are used as a source of enzyme, and the enzyme activity is determined by measuring the conversion of the $^{14}C$-HMG-CoA substrate to $^{14}C$-mevalonic acid.

a. Preparation of Microsomes

Livers are removed from 2-4 cholestyramine-fed, decapitated, Sprague Dawley rats, and homogenized in phosphate buffer A (potassium phosphate, 0.04 M, pH 7.2; KCl, 0.05 M; sucrose, 0.1 M; EDTA, 0.03 M; aprotinin, 500 KI units/ml). The homogenate is spun at 16,000 x g for 15 minutes at 4° C. The supernatant is removed and recentrifuged under the same conditions a second time. The second 16,000 x g supernatant is spun at 100,000 x g for 70 minutes at 4° C. Pelleted microsomes are resuspended in a minimum volume of buffer A (3-5 ml per liver), and homogenized in a glass/glass homogenizer. Dithiothreitol is added (10 mM), and the preparation is aliquoted, quick frozen in acetone/dry ice, and stored at -80° C. The specific activity of the first microsomal preparation was 0.68 nmole mevalonic acid/mg protein/minute.

b. Enzyme Assay

The reductase is assayed in 0.25 ml which contains the following components at the indicated final

concentrations:

| | |
|---|---|
| 0.04 M | Potassium phosphate, pH 7.0 |
| 0.05 M | KCl |
| 0.10 M | Sucrose |
| 0.03 M | EDTA |
| 0.01 M | Dithiothreitol |
| 3.5 mM | NaCl |
| 1% | Dimethylsulfoxide |
| 50-200 µg | Microsomal protein |
| 100 µM | $^{14}C$-[DL]HMG-CoA (0.05 µCi, 30-60 mCi/mmole) |
| 2.7 mM | NADPH (nicotinamide adenine dinucleotide phosphate) |

Reaction mixtures are incubated at 37° C. Under conditions described, enzyme activity increases linearly up to 300 µg microsomal protein per reaction mixture, and is linear with respect to incubation time up to 30 minutes. The standard incubation time chosen for drug studies is 20 minutes, which results in 12-15% conversion of HMG-CoA substrate to the mevalonic acid product. [DL-]HMG-CoA substrate is used at 100 µM, twice the concentration needed to saturate the enzyme under the conditions described. NADPH is used in excess at a level 2.7 times the concentration required to achieve maximum enzyme velocity.

Standardized assays for the evaluation of inhibitors are conducted according to the following procedure. Microsomal enzyme is incubated in the presence of NADPH at 37° C for 15 minutes. DMSO vehicle with or without test compound is added, and the mixture further incubated for 15 minutes at 37° C. The enzyme assay is initiated by adding $^{14}C$-HMG-CoA substrate. After 20 minutes incubation at 37° C the reaction is stopped by the addition of 25 µl of 33% KOH. $^{3}H$-mevalonic acid (0.05 µCi) is added, and the reaction mixture allowed to stand at room temperature for 30 minutes. Fifty µl 5N HCl is added to lactonize the mevalonic acid. Bromophenol blue is added as a pH indicator to monitor an adequate drop in pH. Lactonization is allowed to proceed for 30 minutes at room temperature. Reaction mixtures are centrifuged for 15 minutes at 2800 rpm. The supernatants are layered onto 2 grams AG 1-X8 anion exchange resin (Biorad, formate form) poured in 0.7 cm (id) glass columns, and eluted with 2.0 ml $H_2O$. The first 0.5 ml is discarded, and the next 1.5 ml is collected and counted for both tritium and carbon 14 in 10.0 ml Opti-fluor scintillation fluid. Results are calculated as nmoles mevalonic acid produced per 20 minutes, and are corrected to 100% recovery of tritium. Drug effects are expressed as $I_{50}$ values (concentration of drug producing 50% inhibition of enzyme activity) derived from composite dose response data with the 95% confidence interval indicated.

Conversion of drugs in lactone form to their sodium salts is accomplished by solubilizing the lactone in DMSO, adding a 10-fold molar excess of NaOH, and allowing the mixture to stand at room temperature for 15 minutes. The mixture is then partially neutralized (pH 7.5-8.0) using 1N HCl, and diluted into the enzyme reaction mixture.

2) Cholesterol Synthesis in Freshly Isolated Rat Hepatocytes

Compounds which demonstrate activity as inhibitors of HMG-CoA reductase are evaluated for their ability to inhibit $^{14}C$-acetate incorporation into cholesterol in freshly isolated rat hepatocyte suspensions using methods originally described by Capuzzi et al. (Capuzzi, D.M. and Margolis, S., Lipids, 6:602, 1971).

a. Isolation of Rat Hepatocytes

Sprague Dawley rats (180-220 grams) are anesthetized with Nembutol (50 mg/kg). The abdomen is opened and the first branch of the portal vein is tied closed. Heparin (100-200 units) is injected directly into the abdominal vena cava. A single closing suture is placed on the distal section of the portal vein, and the portal vein is canulated between the suture and the first branching vein. The liver is perfused at a rate of 20 ml/minute with prewarmed (37° C), oxygenated buffer A (HBSS without calcium or magnesium containing 0.5 mM EDTA) after severing the vena cava to allow drainage of the effluent. The liver is additionally perfused with 200 ml of prewarmed buffer B (HBSS containing 0.05% bacterial collagenase). Following perfusion with buffer B, the liver is excised and decapsulated in 60 ml Waymouth's medium allowing free cells to disperse into the medium. Hepatocytes are isolated by low speed centrifugation for 3 minutes at 50xg at room temperature. Pelleted hepatocytes are washed once in Waymouth's medium, counted and assayed for viability by trypan blue exclusion. These hepatocyte enriched cell suspensions routinely show 70-90% viability.

b. $^{14}C$-Acetate Incorporation into Cholesterol

Hepatocytes are resuspended at $5x10^6$ cells per 2.0 ml in incubation medium (IM) [0.02 M Tris-HCl (pH 7.4), 0.1 M KCl, 0.33 mM $MgCl_2$, 0.22 mM sodium citrate, 6.7 mM nicotinamide, 0.23 mM NADP, 1.7 mM glucose-6-phosphate].

Test compounds are routinely dissolved in DMSO or DMSO:$H_2O$ (1:3) and added to the IM. Final DMSO concentration in the IM is ≦ 1.0%, and has no significant effect on cholesterol synthesis.

Incubation is initiated by adding $^{14}C$-acetate (58 mCi/mmol, 2 µCi/ml), and placing the cell suspensions (2.0 ml) in 35 mm tissue culture dishes, at 37° C for 2.0 hours. Following incubation, cell suspensions are transferred to glass centrifuge tubes and spun at 50xg for 3 minutes at room temperature. Cell pellets are resuspended and lysed in 1.0 ml $H_2O$, and placed in an ice bath.

Lipids are extracted essentially as described by Bligh, E. G. and W. J. Dyer, Can. J. Biochem. and Physiol., 37:911, 1959. The lower organic phase is removed and dried under a stream of nitrogen, and the residue resuspended in (100 µl) chloroform:methanol (2:1). The total sample is spotted on silica gel (LK6D) thin-layer plates and developed in hexane: ethyl ether:acetic acid (75:25:1). Plates are scanned and counted using a BioScan automated scanning system. Radiolabel in the cholesterol peak (RF 0.28) is determined and expressed at total counts per peak and as a percent of the label in the total lipid extract. Cholesterol peaks in control cultures routinely contain 800-1000 cpm, and are 9-20% of the label present in the total lipid extract; results compatable with Capuzzi, et al., indicating 9% of extracted label in cholesterol.

Drug effects (% inhibition of cholesterol synthesis) are determined by comparing % of label in cholesterol for control and drug treated cultures. Dose response curves are constructed from composite data from two or more studies, and results are expressed as $I_{50}$ values with a 95% confidence interval.

3) Cholesterol Synthesis in Human Skin Fibroblasts

Compound selectivity favoring greater inhibitory activity in hepatic tissue would be an attribute for a cholesterol synthesis inhibitor. Therefore, in addition to evaluating cholesterol synthesis inhibitors in hepatocytes, these compounds are also tested for their activity as inhibitors of cholesterol synthesis in cultured fibroblasts.

a. Human Skin Fibroblast Cultures

Human skin fibroblasts (passage 7-27) are grown in Eagles' minimal essential medium (EM) containing 10% fetal calf serum. For each experiment, stock cultures are trypsonized to disperse the cell monolayer, counted, and plated in 35 mm tissue culture wells ($5x10^5$ cells/2.0 ml). Cultures are incubated for 18 hours at 37° C in 5% $CO_2$/95% humidified room air. Cholesterol biosynthetic enzymes are induced by removing the serum containing medium, washing the cell monolayers, and adding 1.0 ml of EM containing 1.0% fatty acid free bovine serum albumin, and incubating the cultures an additional 24 hours.

b. $^{14}C$-Acetate Incorporation into Cholesterol

Induced fibroblast cultures are washed with $EMEM_{100}$ (Earle's minimal essential medium). Test compounds are dissolved in DMSO or DMSO:EM (1:3) (final DMSO concentration in cell cultures ≦ 1.0%), added to the cultures, and the cultures preincubated for 30 minutes at 37° C in 5% $CO_2$/95% humidified room air. Following preincubation with drugs, [1-$^{14}C$]Na acetate (2.0 µCi/ml, 58 mCi/mmole) is added, and the cultures reincubated for 4 hours. After incubation, the culture medium is removed, and the cell monolayer (200 µg cell protein per culture) is scraped into 1.0 ml of $H_2O$. Lipids in the lysed cell suspension are extracted into chloroform:methanol as described for hepatocyte suspensions. The organic phase is dried under nitrogen, and the residue resuspended in chloroform:methanol (2:1) (100 µl), and the total sample spotted on silica gel (LK6D) thin-layer plates, V, VO and analyzed as described for hepatocytes.

Inhibition of cholesterol synthesis is determined by comparing the percent of label in the cholesterol peak from control and drug-treated cultures. Results are expressed as $I_{50}$ values, and are derived from composite dose response curves from two or more experiments. A 95% confidence interval for the $I_{50}$ value is also calculated from the composite dose response curves.

A further aspect of the present invention is a pharmaceutical composition consisting of at least one of the compounds of formula I in association with a pharmaceutical vehicle or diluent. The pharmaceutical composition can be formulated employing conventional solid or liquid vehicles of diluents and pharmaceutical additives of a type appropriate to the mode of desired administration. The compounds can be administered by an oral route, for example, in the form of tablets, capsules, granules or powders, or they can be administered by a parenteral route in the form of injectable preparations, such dosage forms containing from 1 to 2000 mg of active compound per dosage, for use in the treatment. Oral administration is preferred. The dose to be administered depends on the unitary dose, the symptoms, and the age and the body weight of the patient.

The compounds of formula I may be administered in a similar manner as known compounds suggested for use in inhibiting cholesterol biosynthesis, such as lovastatin, in mammalian species such as humans, dogs, cats and the like. Thus, the compounds of the invention may be administered in an amount from about 4 to 2000 mg in a single dose or in the form of individual doses from 1 to 4 times per day, preferably 4 to 200 mg in divided dosages of 1 to 100 mg, suitably 0.5 to 50 mg 2 to 4 times daily or in sustained release form.

The following working Examples represent preferred embodiments of the present invention. Unless otherwise indicated, all temperatures are expressed in degrees Centigrade. Flash chromatography was performed on either Merck 60 or Whatmann LPS-I silica gel. Reverse phase chromatography was performed on CHP-20 MCI gel resin supplied by Mitsubishi, Ltd.

As used in the following Examples, the abbreviations "$Et_2O$" "EtOAc", "MeoH" and "EtOH" refer to ethyl ether, ethyl acetate, methanol and ethanol, respectively.

Example 1

(S)-4-[[[4-(4-Fluorophenyl)-1,2-bis(1-methylethyl)]-5-phenyl-1H-pyrrol-3-yl]ethynyl]hydroxyphosphinyl]-3-hydroxybutanoic acid, disodium salt (SQ 34,635)

A. 4-(4-Fluorophenyl)-1,2-bis(1-methylethyl)-5-phenyl-α-(trichloromethyl)-1H-pyrrole-3-methanol, acetate ester

(1) 1-(4-Fluorophenyl)-4-methyl-1-penten-3-one

A mixture of 3-methyl-2-butanone (130.7 mL, 1.22 moles) and p-fluorobenzaldehyde (132.5 mL, 1.22 moles) in methanol (225 mL) was treated with 15% KOH (57 mL) and stirred at room temperature under agron for 24 hours. The reaction mixture was neutralized with glacial acetic acid (7.5 mL), diluted with water (760 mL), then extracted with ethyl acetate (2 times 380 mL). The combined organic extracts were washed with brine (190 mL), dried (anhydrous $MgSO_4$), filtered and evaporated to dryness. The liquid obtained was distilled to give title compound as a light yellow-green syrup (b.pt. 140°, 6-7 mm; 175.88 g, 75.7%) with a consistent $^1$H-NMR spectrum.
TLC: $R_f$ 0.63 (Silica gel:diethyl ether ($Et_2O$): Hexane- 1:4; UV).

(2) 2-(4-Fluorophenyl)-5-methyl-1-phenyl-1,4-hexanedione

A solution of sodium cyanide (588 mg, 12 mmoles) in dry dimethylformamide (30 mL) was heated at 35° (oil bath) for 30 minutes under argon then treated with a solution of benzaldehyde (6.1 mL, 60 mmoles) in dry dimethylformamide (30 mL). The mixture was stirred for 5 minutes at 35°, treated dropwise over a period of 1.5 hours with a solution of Part (1) compound (8.65 g, 45 mmoles) in dry dimethylformamide (50 mL) and stirred at 35° for another hour. The reaction mixture was then diluted with water (100 mL), extracted with dichloromethane (2 x 40 mL) and the combined organic extracts were washed with saturated sodium bicarbonate (100 mL) and water (100 mL), dried (anhydrous $MgSO_4$), filtered and evaporated to dryness. The orange-colored liquid obtained was chromatographed on a silica gel column (Merck), eluting the column with Hexane and $Et_2O$:Hexane (5:95) to give title compound as a thick clear syrup (12.28 g, 91.5%) with consistent $^1$H-NMR and $^{13}$C-NMR spectral data. The product became a waxy solid after standing for a few days at room temperature.
TLC: $R_f$ 0.37 (Silica gel; $Et_2O$:Hexane- 1:4; UV).

(3) 3-(4-Fluorophenyl)-1,5-bis(1-methylethyl)-2-phenyl-1H-pyrrole

A mixture of Part (2) compound (11.34 g, 38 mmoles) and isopropylamine (190 mL, 59 eq.) was cooled down to 0° (ice-salt bath), treated dropwise with glacial acetic acid (150 mL) over a period of 1.0 hour (reaction was exothermic) then warmed up to room temperature. The solid mass was gradually heated up to

reflux conditions, refluxed for 2.0 hours under argon and cooled down to room temperature. The orange semi-solid was suspended in ice-water (400 mL), extracted with ethyl acetate (2 times 900 mL) and the combined organic extracts were washed with saturated sodium bicarbonate (2 times 380 mL), brine (250 mL), dried (anhydrous $MgSO_4$), filtered and evaporated to dryness. The mixture was chromatographed on a silica gel column (Merck) eluting the column with Hexane and $Et_2O$:Hexane (5:95) to give title compound as a solid (11.21 g, 91.8%) with consistent $^1H$-NMR and $^{13}C$-NMR spectral data.

Recrystallization of a small amount from ethyl acetate gave title compound as fine white crystals (m.pt. 149-151°).

TLC: $R_f$ 0.80 (Silica gel; $Et_2O$:Hexane- 1:4; UV, Anisaldehyde).

| Anal. Calc'd for $C_{22}H_{24}FN$: | | | | |
|---|---|---|---|---|
| | C, 82.20; | H, 7.53; | N, 4.36; | F, 5.91 |
| Found: | C, 82.36; | H, 7.76; | N, 4.27; | F, 6.12. |

(4) 4-(4-Fluorophenyl)-1,2-bis(1-methylethyl)-5-phenyl-1H-pyrrole-3-carboxaldehyde

A solution of phosphorus oxychloride (0.43 mL, 4.6 mmoles) in dry acetonitrile (2.6 mL) was cooled down to 0° under argon, treated dropwise with a solution of dimethylformamide (0.34 mL, 4.37 mmoles) in dry acetonitrile, stirred at 0° for 15 minutes then added via cannula to a cooled (0°, ice-salt bath) solution of Part (3) compound (500 mg, 1.56 mmoles) in dry acetonitrile (10 mL). The mixture was warmed up to room temperature and refluxed under argon for 4.0 hours. The solution was then diluted with toluene (16.9 mL), cooled in an ice bath and treated slowly with sodium hydroxide solution (16.9 mL, 1.69 M), keeping the mixture temperature below 25° during the addition. The mixture was warmed up to room temperature, stirred for 1.5 hours and the phases were separated, extracting the aqueous phase with more toluene (17.0 mL). The combined organic extracts were dried (anhydrous $MgSO_4$), filtered and evaporated to dryness. The crude product was chromatographed on a silica gel column (Merck), eluting the column with $Et_2O$:Hexane (1:9) to give title compound as a solid (501 mg, 91.9%).

The product was recrystallized from methanol to give title compound as white crystals (484.5 mg, m.pt. 197-199°).

TLC: $R_f$ 0.22 (Silica gel; $Et_2O$:Hexane- 1:9; UV).

| Anal. Calc'd for $C_{23}H_{24}FNO$: | | | | |
|---|---|---|---|---|
| | C, 79.05; | H, 6.92; | N, 4.01; | F, 5.44 |
| Found: | C, 78.74; | H, 7.03; | N, 3.91; | F, 5.29. |

(5) 4-(4-Fluorophenyl)-1,2-bis(1-methylethyl)-5-phenyl-α-(trichloromethyl)-1H-pyrrole-3-methanol, acetate ester

A solution of Part (4) compound (50 g, 0.143 mole) and dry chloroform (57.3 mL) in dry tetrahydrofuran (1.14 L) was cooled down to -78° (dry ice-acetone), treated dropwise over a period of 30 minutes with 1.0 M lithium bis(trimethylsilyl)amide (160 mL, 0.16 mole) then stirred at -78° for 1.0 hour under argon. The reaction mixture was quenched at -78° with 25% $NH_4Cl$ (400 mL), warmed up to room temperature and diluted with ethyl acetate (1.0 L). The organic phase was separated, washed sequentially with water (570 mL), 5% $KHSO_4$ (570 mL), saturated $NaHCO_3$ (570 mL) and brine (570 mL), dried (anhydrous $MgSO_4$), filtered and evaporated to dryness. The dark syrup obtained was dried in vacuo, dissolved in a mixture of dry pyridine (570 mL) and acetic anhydride (858 mL) then stirred overnight at room temperature under argon. TLC indicated that the reaction was not complete so the mixture was heated at 60° (oil bath) for 4.0 hours, cooled, evaporated to dryness and dried in vacuo. The dark brown solid (75.99 g) was dissolved in ethyl acetate (2.0 L) and filtered through a silica gel pad, washing the pad well with ethyl acetate (1.0 L). The light brown filtrate was evaporated to dryness and the solid obtained triturated with methanol to give title compound as tan crystals (69.185 g, 94.7%). A sample recrystallized from EtOAc-hexane had mp 203-205°C (decomp.)

| Anal. for $C_{26}H_{27}Cl_3FNO_2$: | | | | | |
|---|---|---|---|---|---|
| Calc'd: | C, 61.13; | H, 5.33; | N, 2.74; | F, 3.72; | Cl, 20.82 |
| Found: | C, 61.24; | H, 5.24; | N, 2.71; | F, 3.84; | Cl, 20.54 |

$^1$H-NMR Spectrum # 405762 (400 MHz, $CDCl_3$): δ

| | |
|---|---|
| 1.08 | (broad singlet, 3 H, ------, $CH_3$ of N-$CH(CH_3)_2$) |
| 1.35 | (d, 3 H, -----, $CH_3$ of N-$CH(CH_3)_2$) |
| 1.44 | (d, 6 H, J=7 Hz, $CH_3$ of $C_2$-$CH(CH_3)_2$) |
| 2.24 | (s, 3 H, ----, -CO-$CH_3$) |
| 4.15 | (m, 1 H, ----, N-CH-$(CH_3)_2$) |
| 4.65 | (septet, 1 H, ----, $C_2$-CH-$(CH_3)_2$) |
| 6.57 | (broad singlet, 1 H, ------, $C_3$-CH(OAc)$CH_3$) |
| 6.79-7.19 | (m, 8 H, -----, aromatic protons) |

B. 3-(2,2-Dichloroethenyl)-4-(4-fluorophenyl)-1,2-bis(1-methylethyl)-5-phenyl-1H-pyrrole

A mixture of Part A compound (79.7 g 0.156 mole), lead(II) chloride (9.22 g, 32.8 mole) and dry dimethylformamide (1.6 L) under nitrogen was treated with 5.16 g (0.191 mole) of aluminum foil (cut into 1 cm x 4 cm pieces and crumpled slightly). Stirring and heating to 60°C gave initially an amber clear solution containing unreacted aluminum and dark lumps of lead-aluminum alloy, then, after 1.5 hours, a slurry of white solid, unreacted aluminum, and alloy. A sample diluted with ethyl acetate showed the absence of starting material by TLC (20% ethyl acetate in hexane). The mixture was diluted to 3L with ethyl acetate and filtered on a celite pad. The cake was washed with 1L of ethyl acetate and then with 1L of methylene chloride. The filtrates were combined and washed with 500 mL of 10% sodium bisulfate, 2 times 500 mL of water, and 500 mL of brine. Drying over magnesium sulfate and evaporation in vacuo gave a slurry. Trituration with hexane gave a wet, beige solid. Washing this solid with ether gave a white solid which, on drying in vacuo gave 54.19 g of title compound.

The mother liquors were evaporated in vacuo, finally on a vacuum pump, to give a residue which, on trituration with diethyl ether and drying gave another 4.55 g of homogeneous title compound, for a total yield of 58.74 g (90.2%). Recrystallization of 77 mg of product from $Et_2O$-hexane (1:5) gave white crystals of title compound (48.9 mg, m.pt. 182-3°C).
TLC: $R_f$ 0.72 (Silica gel; EtOAc-hexane - 1:4; UV).

| Anal. Calc'd for $C_{24}H_{24}Cl_2FN$: | | | | | |
|---|---|---|---|---|---|
| | C, 69.23; | H, 5.87; | N, 3.36; | F, 4.56; | Cl, 17.03 |
| Found: | C, 69.07; | H, 5.79; | N, 3.90; | F, 4.67; | Cl, 16.86. |

C. 3-Ethynyl-4-(4-fluorophenyl)-1,2-bis(1-methylethyl)-5-phenyl-1H-pyrrole

A solution of Part B compound (58.74 g, 140.7 mmoles) in 900 mL of dry tetrahydrofuran was added over 3/4 hour to a mixture of 195 mL (312 mmoles) of 1.6M n-butyl lithium (in hexanes) and 250 mL of dry tetrahydrofuran at -78°C under nitrogen. After stirring at -78°C for 1/2 hour, TLC (hexane/acetone, 4/1) showed the presence of considerable Part B compound. Two additional 50 mL portions of n-butyl lithium were added with stirring for 1/2 hour after each addition. A trace of starting material remained after the second addition. Another 20 mL of n-butyl lithium was added followed, after 10 minutes, by 500 mL of saturated ammonium chloride over 5 minutes. The mixture was warmed to room temperature over 1 hour with the aid of a water bath, then was diluted to 3L with ethyl acetate. The layers were separated and the organic phase was dried over sodium sulfate and evaporated to a solid. Trituration with hexane gave title compound as an off-white solid, 49.15 g (theo. = 48.75 g) after drying in vacuo, which contained 6% of unreacted Part A compound as determined by NMR.

D. (S)-4-(Chloromethoxyphosphinyl)-3-[[(1,1-dimethylethyl)diphenylsilyl]oxy]butanoic acid

(S)-4-(Hydroxymethoxyphosphinyl)-3-[[(1,1-dimethylethyl)diphenylsilyl]oxy]butanoic acid, methyl ester,

dicyclohexylamine (1:) salt, prepared as described in Example 22 of U.S. Patent No. 4,904,646 (126 g, 198 mmoles) was shaken with excess 10% sodium bisulfate and 2L of ethyl acetate. The layers were separated and the organic was dried over magnesium sulfate and evaporated. The free acid was dissolved in 500 mL of dichloromethane and was treated with 90 mL of trimethylsilyldiethylamine. After 18 hours under nitrogen, the volatiles were evaporated in vacuo and the residue azeotroped twice with 250 mL of toluene. The resulting oil was pumped in vacuo for 3/4 hour, then was taken up in 500 mL dichloromethane, treated with 2 mL of dry dimethylformamide and chilled to 0 °C. Oxalyl chloride (100 mL of a 2M solution in dichloromethane) was added over 1/2 hour. Stirring was continued for 3/4 hour at 0 °C, then for 1.5 hours at 20 °C. The volatiles were removed in vacuo and the residue was azeotroped with 250 mL of toluene. The resulting semisolid was slurried in a second 250 mL portion of dry toluene, filtered on a dry sintered glass funnel under nitrogen, and the filtrate evaporated to an oil in vacuo. The title ester in the form of a clear brown-red oil was dissolved in 300 mL of dry tetrahydrofuran and the solution chilled to -70 °C under nitrogen.

E. (s)-3-[[(1,1-Dimethylethyl)diphenylsilyl]oxy]-4-[[[4-(4-fluorophenyl)-1,2-bis(1-methylethyl)-5-phenyl-1H-pyrrol-3-yl]ethynyl]methoxyphosphinyl]butanoic acid, methyl ester

A solution of Part C compound (40 g, containing 107 mmoles of Part C compound and 7 mmoles of Part B compound in 600 mL of dry tetrahydrofuran was chilled to -70 °C under nitrogen and treated with 75 mL of 1.6M n-butyl lithium in hexanes (120 mmoles). After stirring at -70 °C for 20 minutes, the clear amber solution was cannulated into the cold solution of Part D compound over 20 minutes. The mixture was stirred at -70 °C for 30 minutes, then was quenched by the addition of 250 mL of saturated ammonium chloride over 5 minutes. The mixture was allowed to warm up over 1 hour, then was partitioned between water and 2L of ethyl acetate. The organic phase was washed with water (2 times with 1L) and brine (0.5L), dried over sodium sulfate and evaporated to an oil. This material was dissolved in 6% isopropanol in hexane and chromatographed in the same solvent on 8L of K-60 silica gel (Merck). Homogeneous fractions (10L) were pooled and evaporated to give 101.4 g (80.7%) of title compound as a thick oil.

F. (S)-4-[[[4-(4-Fluorophenyl)-1,2-bis(1-methylethyl)-5-phenyl-1H-pyrrol-3-yl]ethynyl]hydroxyphosphinyl]-3-hydroxybutanoic acid, disodium salt

A solution of Part E compound (101.4 g, 130 mmoles) in 700 mL of tetrahydrofuran under nitrogen was treated with 46 g of glacial acetic acid followed by 600 mL of a 1M solution of tetrabutylammonium fluoride in tetrahydrofuran. After stirring at ambient temperatures for 20 hours, TLC (hexane/acetone, 1/1) showed complete reaction. The mixture was diluted to 2.5L with ethyl acetate, chilled in an ice bath, and treated with stirring over 15 minutes with 500 mL of 1.5% aqueous hydrochloric acid. The layers were separated, and the organic layer was diluted with another 1L of ethyl acetate and washed with 1L of 1.5% HCl, 2 times with 1L of water, and 0.5L of brine. The aqueous washes were reextracted with ethyl acetate. Drying of the organic extracts and evaporation in vacuo gave 105 g of a thick oil. This oil was dissolved in 1.1L of methanol and treated with 560 mL of 10% aqueous sodium hydroxide. The mixture was heated on a steam cone for 15 minutes then was allowed to come to room temperature over 1 hour. The mixture was cooled in ice and treated carefully with enough 10% HCl to bring the pH down to 1.5. This cold mixture was extracted promptly with ethyl acetate (2L) and the extracts washed quickly with 0.5L of cold 3% HCl and 2 times with 1L of water. Drying over sodium sulfate for 15 minutes and evaporation at 15 °C in vacuo gave an oil which was promptly dissolved in 500 mL of methanol and treated with enough 10% sodium hydroxide to bring the pH to 8.6. The volatiles were evaporated in vacuo to give a solid. This was triturated with acetonitrile to give a near-white solid. This material was filtered and then taken up in 1L of acetonitrile/water (1/1) with warming on a steam cone. The mixture was diluted while hot with hot acetonitrile (2L) whereupon a massive cotton-like precipitate formed. This slurry was cooled nearly to room temperature, filtered, and washed with 10% water in acetonitrile and finally acetonitrile. Two additional crops were obtained on further dilution of the filtrates with acetonitrile. The last crop was recrystallized in a similar fashion to obtain material of purity similar to the first two crops by TLC (dichloromethane/methanol/acetic acid, 8/1/1). Then materials of similar purity were combined and recrystallized by dissolving in 800 mL of acetonitrile/water (1/1) and dilution of 3L with hot acetonitrile. Filtering while still warm and washing with 10% water in acetonitrile followed by acetonitrile gave a white solid. This was dried in vacuo for two days at 20 °C to give 40.57 g of title product.

Alternative Preparation of 4-(4-Fluorophenyl)-1,2-bis(1-methylethyl)-5-phenyl-1H-pyrrole-3-carboxaldehyde [Example 1A, Part(4)]

A. β-(4-Fluorophenyl)-α-(2-methyl-1-oxopropyl)-γ-oxobenzenebutanoic acid, ethyl ester

(1) 2-[(4-Fluorophenyl)methylene]-4-methyl-3-oxopentanoic acid, ethyl ester

A mixture of p-fluorobenzaldehyde (7.78 mL, 72.5 mmoles), isobutyryl acetate (11.69 mL, 72.4 mmoles), piperidine (0.72 mL) and glacial acetic acid (125 μL) in dry benzene (45 mL) was refluxed under argon for 6.0 hours using a Dean-Starke trap. The reaction mixture was cooled, extracted with diethyl ether (50 mL) and the organic phase was washed successively with 2% HCl (18 mL), 5% sodium bicarbonate (20 mL) and brine (15 mL). The organic solution was dried (anhydrous $MgSO_4$), filtered and evaporated to remove as much of the solvent as possible. The orange syrup obtained was distilled to give title compound (16.952 g, 87.0%, b.pt. 155-157°, 1.5 mm.).
TLC: $R_f$ 0.53 (Silica gel; EtOAc:Hexane- 1:4; UV).

(2) β-(4-Fluorophenyl)-α-(2-methyl-1-oxopropyl)-γ-oxobenzenebutanoic acid, ethyl ester

A mixture of Part (1) compound (14.59 g, 55.4 mmoles), 3-benzyl-5-(2-hydroxyethyl)-4-methylthiazolium chloride (4.484 g, 0.3 eq.) and triethylamine (5.4 mL, 0.7 eq.) was treated with benzaldehyde (1.9 mL), heated up to 70° (oil bath) under argon and stirred at 70° for 1.0 hour. The addition of benzaldehyde was repeated three more times (1.9 mL each), heating the mixture for 1.0 hour after each addition. The reaction mixture was cooled down to room temperature, diluted with ethyl acetate (800 mL) and washed successively with 5% $KHSO_4$ (150 mL), saturated sodium bicarbonate (150 mL) and brine (150 mL). The organic solution was dried (anhydrous $MgSO_4$), filtered, evaporated to dryness and dried *in vacuo*. The crude product was combined with previous runs (881.6 mg and 236.5 mg) and chromatographed on a silica gel column (Merck), eluting the column with $Et_2O$:Hexane mixtures (5:95; 1:9) to give title compound as a light yellow thick syrup (20.694 g, 98.3%).
TLC: $R_f$ 0.22 (Silica gel; $Et_2O$:Hexane- 15:85; UV).

B. 4-(4-Fluorophenyl)-1,2-bis(1-methylethyl)-5-phenyl-1H-pyrrole-3-carboxylic acid, ethyl ester

A solution of Part A compound (2.5 g, 6.77 mmoles) in dry toluene (27 mL) was cooled down to 0° (ice-salt bath) under argon and treated with isopropylamine (3.04 mL, 5.28 eq.) and 1.0 M $TiCl_4$ (6.77 mL, 1 eq.). The mixture was warmed up to room temperature, stirred for 24 hours, cooled back down to 0° and treated once more with isopropylamine (1.9 mL, 3.33 eq.) and 1.0 M $TiCl_4$ (4.1 mL, 0.6 eq.). The mixture was warmed up to room temperature and stirred for another 24 hours, diluted with ethyl acetate (375 mL) and filtered through a celite pad in a millipore unit, washing the pad well with ethyl acetate (175 mL). The combined organic solutions were washed with 5% $KHSO_4$ (2 x 75 mL), saturated $NaHCO_3$ (50 mL), brine (75 mL), dried (anhydrous $MgSO_4$), filtered, evaporated to dryness and dried *in vacuo*. The crude product mixture (2.589 g) was chromatographed on silica gel column (Merck), eluting the column with Hexane and $Et_2O$:Hexane mixtures (5:95; 1:9) to give title compound as a light gray solid (1.217 g, 45.8%).

Recrystallization of 100 mg of the product from $Et_2O$:Hexane (1:1) gave title compound as a white solid (36.4 mg, m.pt. 152-153°).
TLC: $R_f$ 0.60 (Silica gel; $Et_2O$:Hexane- 1:4; UV).

| Anal. Calc'd for $C_{25}H_{28}FNO_2$: | | | | |
|---|---|---|---|---|
| | C, 76.31; | H, 7.17; | N, 3.56; | F, 4.83 |
| Found: | C, 76.23; | H, 7.22; | N, 3.57; | F, 4.75. |

C. 4-(4-Fluorophenyl)-1,2-bis(1-methylethyl)-5-phenyl-1H-pyrrole-3-methanol

A solution of Part B compound (1.166 g, 2.96 mmoles) in dry tetrahydrofuran (12.5 mL) was added dropwise to a cooled (0°, ice-salt bath) suspension of lithium aluminum hydride (169 mg, 4.45 mmoles) in dry tetrahydrofuran (2.5 mL), warmed up to room temperature and stirred under argon for 24 hours. The reaction mixture was cooled back down to 0°, quenched by the sequential addition of water (0.17 mL), 15% NaOH (0.17 mL) and water (0.51 mL), stirred for 15 minutes then warmed up to room temperature. The reaction mixture was diluted with ethyl acetate (25 mL) and filtered through a celite pad in a millipore unit, washing the pad well with ethyl acetate (15 mL). The clear filtrate was evaporated to dryness and dried *in*

vacuo to give title compound as a solid (1.076 g, 100%).

Recrystallization of the crude product from $Et_2O$:Hexane (1:4) gave title compound as a white solid (60 mg, m.pt. 139-140°).

TLC: $R_f$ 0.15 (Silica gel; $Et_2O$:Hexane- 1:4; UV).

| Anal. Calc'd for $C_{23}H_{26}FNO$: | | | | |
|---|---|---|---|---|
| | C, 78.60; | H, 7.46; | N, 3.99; | F, 5.41 |
| Found: | C, 78.43; | H, 7.62; | N, 4.00; | F, 5.29. |

D. 4-(4-Fluorophenyl)-1,2-bis(1-methylethyl)-5-phenyl-1H-pyrrole-3-carboxaldehyde

A solution of 97% 4-methylmorpholine N-oxide (461.6 mg, 3.83 mmoles, 1.8 eq.) was dissolved in dry dichloromethane (15 mL), dried (anhydrous $MgSO_4$) and filtered. The solution was added to a mixture of Part C compound (750 mg, 2.13 mmoles) and 4 Å sieves (1.25 g, oven-dried), stirred for 5 minutes under argon, then treated with tetraisopropylammonium peruthinate (TPAP (40.6 mg, 0.115 mmole). The reaction mixture was stirred for 30 minutes, diluted with diethyl ether (175 mL) and filtered through a celite pad in a millipore unit, washing the pad well with diethyl ether (90 mL) and $CH_2Cl_2$ (15 mL), and the clear light brown filtrate was evaporated to dryness and dried in vacuo.

The crude product was chromatographed on a silica gel column (Merck), eluting the column with Hexane and $Et_2O$:Hexane (5:95) to give title compound as a solid (663.9 mg, 89.2%).

Recrystallization of 100 mg from diethyl ether gave title compound as a white solid (74.3 mg, m.pt. 196-198°).

TLC: $R_f$ 0.40 (Silica gel; $Et_2O$):Hexane- 1:4; UV).

| Anal. Calc'd for $C_{23}H_{24}FNO$: | | | | |
|---|---|---|---|---|
| | C, 79.05; | H, 6.92; | N, 4.01; | F, 5.44 |
| Found: | C, 78.95; | H, 6.87; | N, 3.85; | F, 5.40. |

Example 2

(S)-4-[[[4-(4-Fluorophenyl)-1,2-bis(1-methylethyl)-5-phenyl-1H-pyrrol-3-yl]-ethynyl]hydroxyphosphinyl]-3-hydroxybutanoic acid, dilithium salt (SQ 34,346)

A. (S)-4-[[[4-(4-Fluorophenyl)-1,2-bis(1-methylethyl)-5-phenyl-1H-pyrrol-3-yl]-ethynyl]methoxyphosphinyl]-3-hydroxybutanoic acid, methyl ester

A solution of Example 1, Part E compound (867 mg, 1.11 mmoles) in dry tetrahydrofuran (6.5 mL) was treated successively with glacial acetic acid (0.25 mL, 4.26 mmoles, 4 eq) and 1.0 M $(n\text{-}C_4H_9)_4NF$ (3.32 mL, 3.32 mmoles, 3 eq) and stirred under argon at room temperature for 48 hours. The reaction mixture was diluted with water (6.2 mL), extracted with EtOAc (2 x 40 mL), and the combined organic extracts were washed with saturated $NaHCO_3$ (8.1 mL), 1.0 N HCl (3 x 3.4 mL) and brine (6.0 mL). The solution was then dried (anhydrous $MgSO_4$), filtered, evaporated to dryness and dried in vacuo.

The crude product (958 mg) was dissolved in dry diethyl ether (10 mL), cooled down to 0° (ice-salt bath), treated with excess diazomethane in diethyl ether and stirred at 0° for 1.0 hour under argon. The reaction mixture was quenched by the dropwise addition of glacial acetic acid (0.2 mL), evaporated to dryness and dried in vacuo. The crude product was chromatographed on a silica gel column (Merck, 50:1), eluting the column with Acetone:Hexane mixtures (1:9; 1:4; 1:2), and the desired fractions were combined and evaporated to dryness to give title compound as a syrup (516.3 mg, 86.2%) with consistent $^1$H-NMR and $^{13}$C-NMR spectral data.

TLC: $R_f$ 0.38 (Silica gel; Acetone:Hexane- 1:1; UV).

B. (S)-4-[[[4-(4-Fluorophenyl)-1,2-bis(1-methylethyl)-5-phenyl-1H-pyrrol-3-yl]ethynyl]hydroxyphosphinyl]-3-hydroxybutanoic acid, dilithium salt (SQ 34,346)

A solution of Part H compound (617 mg, 1.14 mmoles) in dioxane (19.8 mL) was treated with 1.0 N LiOH (4.0 mL, 3.5 eq) and stirred at room temperature under argon for 20 hours and at 50° (oil bath) for 3.0 hours. The reaction mixture was evaporated to dryness and dried in vacuo. The crude product was chromatographed on an HP-20 column (1" x 7"), eluting the column with steam-distilled water (500 mL) and aqueous $CH_3OH$ (10%, 500 mL; 20%, 500 mL; 50%, 500 mL). The desired fractions were combined, evaporated to dryness and dried in vacuo. The solid product was dissolved in steam-distilled water and lyophilized to give title product as a fluffy white solid lyophilate (444.8 mg, 69.9%).
TLC: $R_f$ 0.35 (Silica gel; i-$C_3H_7OH$:$NH_4OH$:$H_2O$-8:1:1; UV).

| Anal. Calc'd for $C_{28}H_{29}FLi_2NO_5P$ x 1.928 moles $H_2O$ (Eff. Mol Wt. = 558.102): | | | | | |
|---|---|---|---|---|---|
| | C, 60.38; | H, 5.93; | N, 2.51; | F, 3.40; | P, 5.55 |
| Found: | C, 60.38; | H, 6.02; | N, 2.39; | F, 3.79; | P, 5.68. |

IR(KBr) # 74239 (2159 $cm^{-1}$, C≡C; 1592 $cm^{-1}$, C=O of $COO^-$).
MS (FAB/SIMS) $(M+H)^+$ = 524.
$^1$H-NMR Spectrum (270 MHz, $CD_3OD$): δ

1.43 (d, 6H, J=7 Hz)
1.56 (d, 6H, J=7 Hz)
1.85 (m, 2H)
2.32 (dd, 1H, J=8, 15 Hz)
2.44 (dd, 1H, J=4, 15 Hz)
3.43 (septet, 1H, J=7 Hz)
4.39 (m, 2H)
6.82-7.35 (m, 9H)

Examples 4 to 10

Following the procedures as outlined heretofore and as described in the previous working Examples, the following additional compounds may be prepared.

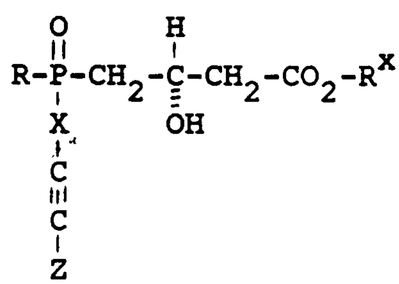

| Ex. No. | R | Z | $R^x$ |
|---|---|---|---|
| 4. | NaO | 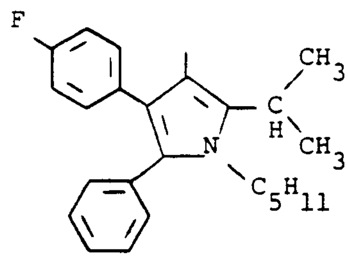 | Na |
| 5. | NaO | 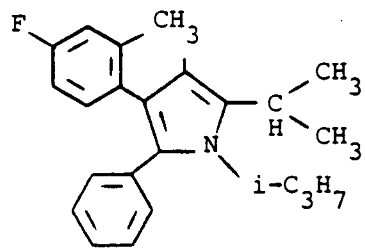 | Na |
| 6. | LiO | 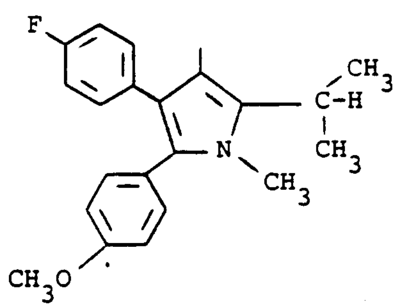 | Li |
| 7. | LiO | 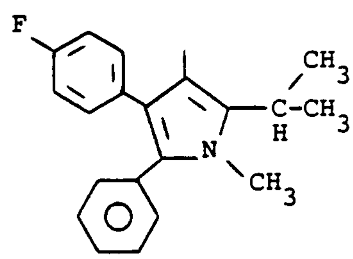 | Li |

| Ex. No. | R | Z | $R^x$ |
|---|---|---|---|
| 8. | NaO | 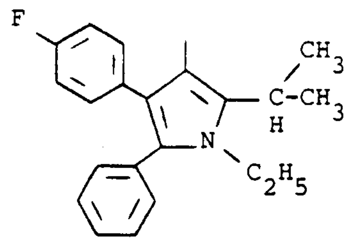 | Na |
| 9. | $CH_3O$ | 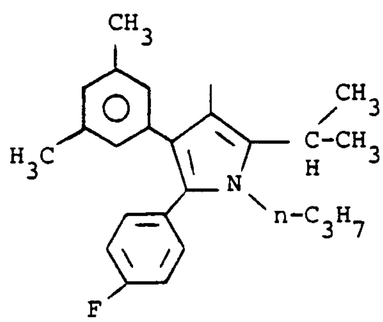 | H |
| 10. | $CH_3O$ | 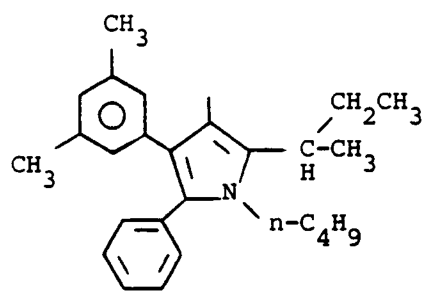 | $NH_4$ |
| 11. | LiO | 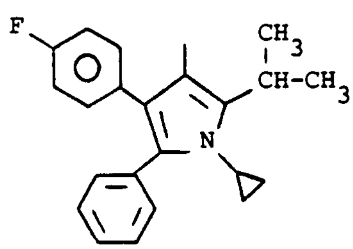 | Li |

| Ex. No. | R | Z | $R^x$ |
|---|---|---|---|
| 12. | LiO | 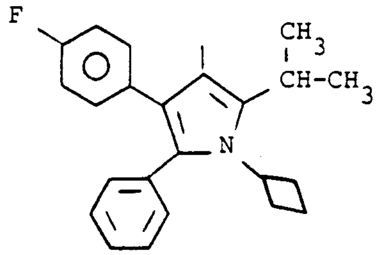 | Li |
| 13. | LiO | 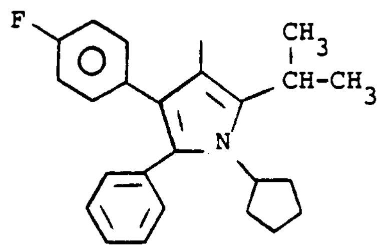 | Li |

Example 11

SQ 34,346 was tested in the following in vitro test:

1) "Rat Hepatic HMG-CoA Reductase" -test 1) described on pages 37 to 40;
2) Cholesterol Synthesis in Freshly Isolated Rat Hepatocytes - test 2) described on pages 40 to 42;
3) Cholesterol Synthesis in Human Skin Fibroblasts - Test 3) described on pages 42 to 44.

The results obtained were as follows:

1) Rat Hepatic HMG-CoA Reductase test Reductase $I_{50}$ = 0.0006 µM
2) Cholesterol Synthesis in Freshly Isolated Rat Hepatocytes test - Hepatocytes $I_{50}$ = 0.078 µM
3) Cholesterol Synthesis in Human Skin Fibroblasts - Fibroblasts $I_{50}$ = 2.5 µM.

The above in vitro test results show that the test compound SQ 34,346 is a potent inhibitor of cholesterol biosynthesis.

Example 12

SQ 34,346 in oral or IV dosage form was tested in vivo for its effect on cholesterol biosynthesis in rats ($^{14}C$-Acetate Model).

The following in vivo tests employed a rat model system to determine the in vivo cholesterol synthesis inhibitory activity of compounds which show cholesterol synthesis inhibition in whole cell systems (freshly isolated rat hepatocytes and cultured human skin fibroblasts), and which are potent inhibitors of HMG CoA reductase. The method used in this model is designed to determine whether test compounds inhibit cholesterol synthesis in vivo, and is not a direct assay for the ability of test compounds to lower blood cholesterol levels. However, compounds active in this in vivo model are expected to demonstrate hypocholesterolemic activity in appropriate animal systems (where LDL is a major cholesterol carrying blood component), since other drugs which inhibit cholesterol synthesis in vivo lower circulating cholesterol levels through inhibition of synthesis and coordinate up-regulation of hepatic LDL receptors.

Method:

The method used for iv and po drug testing were adapted from a procedure originally described by

Sandoz (U.S. Patent No. 4,613,610, Sept. 23, 1986, and Int'l Publication No. WO 86/00367, Derwent Nos. 86271640 and 8628274). The method was originally designed around the observation that cholesterol synthesis in rats is greatest at the mid-dark point of the (12h light/12h dark) diurnal light cycle. Male Sprague/Dawley rats (200-300 grams) were adapted to a reverse light cycle for 7-10 days, and fed Purina rat chow (#5001) ad libitum. In order to measure cholesterol synthesis [1-$^{14}$C]-sodium acetate (1-3 mCi/mmole) 25 μCi/100 grams body weight was injected ip two hours before the mid-dark point in the diurnal cycle. Two hours after the mid-dark point animals were anesthetized with ketamine/xylazine and bled into EDTA treated centrifuge tubes from the abdominal aorta. Plasma was obtained by centrifugation at 1100 x g for 10 minutes. One mL plasma samples were aliquoted and either processed directly or frozen at -20 °C.

For iv testing, the salt forms of test compounds were routinely dissolved in saline and injected iv into the tail vein 5 minutes before $^{14}$C-acetate injection. For po testing drugs were dissolved in saline (or suspended in 0.05% CMC in saline) and given 30 minutes before $^{14}$C-acetate injection. These dosing procedures were routinely followed unless otherwise noted. Cholesterol synthesis was measured by determining the level of $^{14}$C-nonsaponifiable lipid present in one mL of plasma; the method used is a modification of the method described by Dugan, et al, (Dugan, R.E., L.L. Slakey, A.V. Briedis, and J. Porter, Arch. Biochem. Biophys., 152:21 (1972)). One mL physiological saline was added to one mL plasma; followed by the addition of 5.0 mL 10% KOH in absolute ethanol. Samples were mixed and saponified at 75 °C for one hour. After cooling, approximately 0.02 μCi (44,000 dpm) [1,2-$^{3}$H] Cholesterol (40-60 Ci/mmole) was added to each sample. Samples were extracted once with 5 mL petroleum ether, and the organic phase backwashed once with 5 mL saline. This extraction procedure resulted in 50-90% recovery of the added $^{3}$H-cholesterol internal standard. The extracts were dryed in glass vials and the residue resuspended in 0.5 mL chloroform/methanol (2:1). Samples were counted for both $^{3}$H and $^{14}$C in 10 mL Optiflour scintillation fluid using a Beckman LS3801 counter programed for double label counting. The $^{3}$H-cholesterol internal standard percent recovery value form each sample was used to correct each sample to 100% recovery of $^{14}$C-cholesterol.

Due to the variability in cholesterol synthesis among individual animals it is necessary to include 4-5 rats per treatment group. Results are expressed as percent inhibition of cholesterol synthesis (or % inhibition of plasma $^{14}$C-nonsaponifiable lipid) and are derived by comparing average $^{14}$C-nonsaponifiable plasma lipid values per mL plasma from control and drug-treated animal groups. Percent inhibition is plotted relative to the log drug dose, and a linear best fit regression line is determined for each experiment. An $ED_{50}$ value (level of drug required to suppress cholesterol synthesis in vivo by 50 percent) is calculated for each experiment. For drugs tested in more than one experiment a mean $ED_{50}$ ± S.E.M. is determined and reported. Projected $ED_{50}$ values are not included in calculations of the mean $ED_{50}$ values.

The following results were obtained

In Vivo

$ED_{50}$ = 0.05 mg/kg, i.v.
$ED_{50}$ = 1.13 mg/kg p.o.

The above in vivo test results show that the subjest compound (SQ 34,346) is a potent inhibitor of cholesterol biosynthesis when administered intravenously and perorally.

## Claims

**1.** A compound having the structure

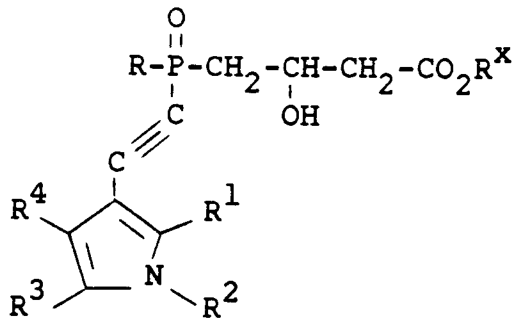

wherein R is OH or lower alkoxy;

$R^x$ is H or lower alkyl, including salts thereof, where in such salts $R^x$ includes a salt moiety or each of $R^x$ and R includes a salt moiety;

$R^1$ is lower alkyl;

$R_2$ is lower alkyl;

$R^3$ is phenyl or phenyl substituted with one, two or three lower alkyl, lower alkoxy (except t-butoxy) or halogen groups;

$R^4$ is phenyl or phenyl substituted with one, two or three lower alkyl, lower alkoxy (except t-butoxy) or halogen groups.

**2.** The compound as defined in Claim 1 wherein R is OH.

**3.** The compound as defined in Claim 1 wherein $R^x$ is H or includes a salt moiety.

**4.** The compound as defined in Claim 1 wherein $R^3$ is phenyl and $R^4$ is halosubstituted-phenyl.

**5.** The compound as defined in Claim 1 wherein $R^1$ is isopropyl and $R^2$ is isopropyl.

**6.** The compound as defined in Claim 1 having the formula

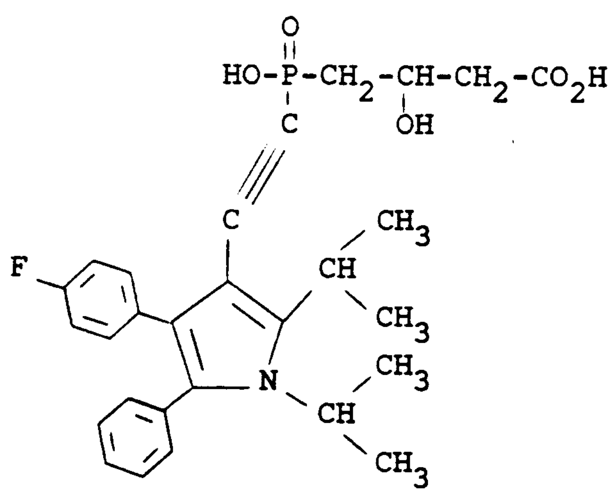

including the disodium or dilithium salts thereof.

**7.** The compound as defined in Claim 1 having the name (S)-4-[[[4-(4-fluorophenyl)-1,2-bis(1-methylethyl)-5-phenyl-1H-pyrrol-3-yl]ethynyl]hydroxyphosphinyl]-3-hydroxybutanoic acid, disodium salt or the corresponding dilithium salt.

**8.** A hypocholesterolemic or hypolipemic composition comprising a compound as defined in Claim 1 and a pharmaceutically acceptable carrier therefor.

**9.** The composition as defined in Claim 8 in oral dosage form.

**10.** Use of a compound as defined in Claim 1 for the preparation of a pharmaceutical composition for the inhibition of cholesterol biosynthesis.

**11.** Use as defined in Claim 10 wherein said pharmaceutical composition is administered in oral dosage form.

**12.** A compound of the formula

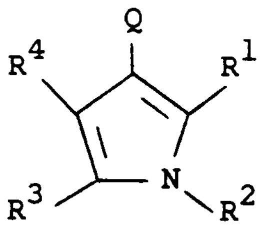

wherein Q is

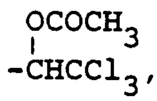

$-CH=CCl_2$ or $-C\equiv CH$.

**13.** A compound of the formula

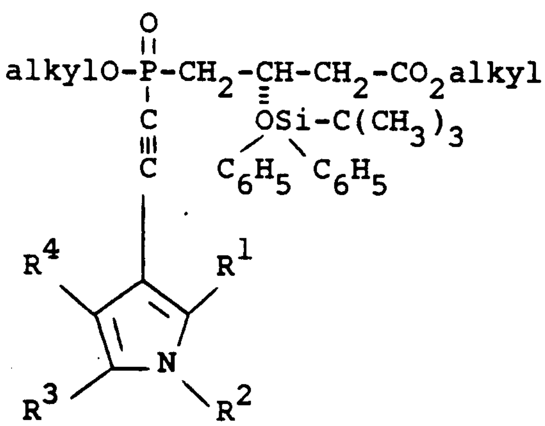

including all stereoisomers thereof.

**14.** A process for preparing a compound of the structure

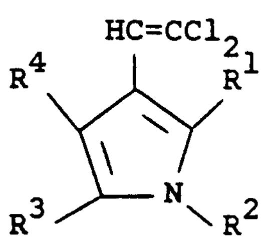

wherein $R^1$ is lower alkyl;
$R^2$ is lower alkyl;
$R^3$ is phenyl or phenyl substituted with one, two or three lower alkyl, lower alkoxy (except t-butoxy) or halogen groups;
$R^4$ is phenyl or phenyl substituted with one, two or three lower alkyl, lower alkoxy (except t-butoxy) or halogen groups, which comprises treating a cooled solution of an aldehyde of the structure

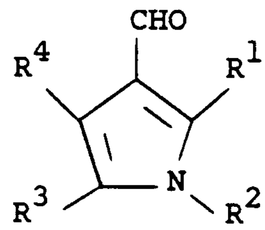

in dry inert organic solvent and dry chloroform with lithium bis(trimethylsilyl)amide under an inert atmosphere, treating the resulting reaction product with acetic anhydride and pyridine, under an inert atmosphere, to form a trichloride compound of the structure

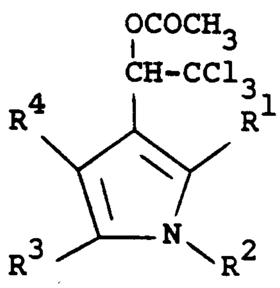

and treating the trichloride with lead chloride and aluminum in dry inert organic solvent under an inert atmosphere to form the dichloride.

**15.** A method for preparing a compound having the structure

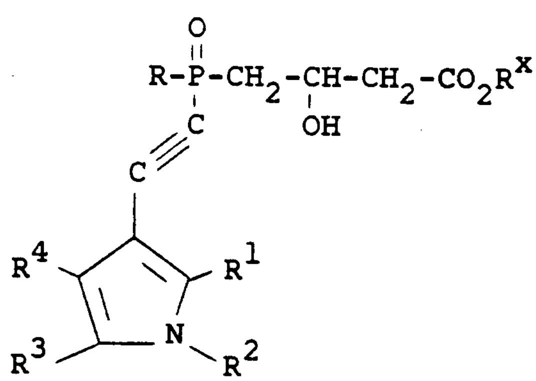

wherein R is OH or lower alkoxy;
$R^x$ is H or lower alkyl, including salts thereof, wherein such salts $R^x$ include a salt moiety or each of $R^x$ and R includes a salt moiety;
$R^1$ is lower alkyl;
$R^2$ is lower alkyl;
$R^3$ is phenyl or phenyl substituted with one, two or three lower alkyl, lower alkoxy (except t-butoxy) or halogen groups;
$R^4$ is phenyl or phenyl substituted with one, two or three lower alkyl, lower alkoxy (except t-butoxy)

or halogen groups, which comprises treating a cooled solution of an aldehyde of the structure

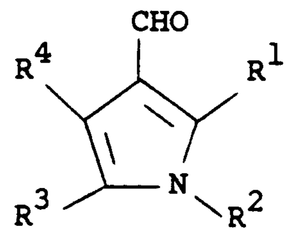

in dry inert organic solvent and dry chloroform with lithium bis(trimethylsilyl)amide under an inert atmosphere, treating the resulting reaction product with acetic anhydride and pyridine, under an inert atmosphere, to form a trichloride compound of the structure

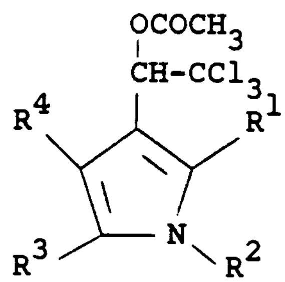

treating the trichloride with lead chloride and aluminum in dry inert organic solvent under an inert atmosphere to form a dichloride of the formula

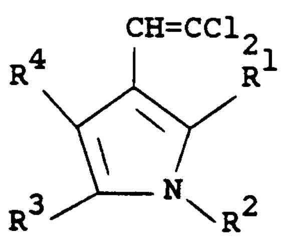

reacting the dichloride with a strong base to form the acetylene compound of the formula

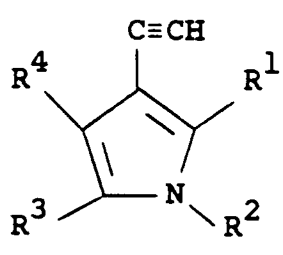

condensing the acetylene compound with a phosphonochloridate of the formula

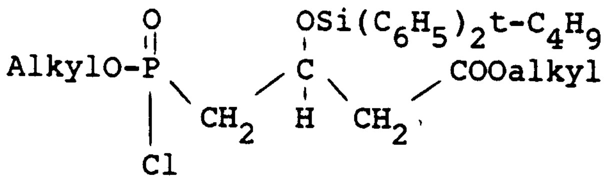

in the presence of base to form the ester of the formula

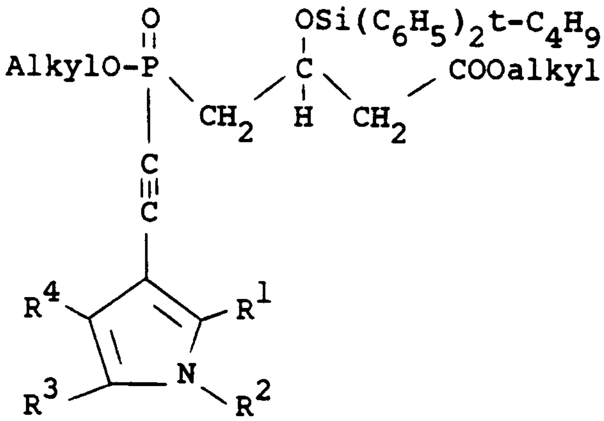

reacting the above ester with a silyl ether cleaving agent to form the ester of the formula

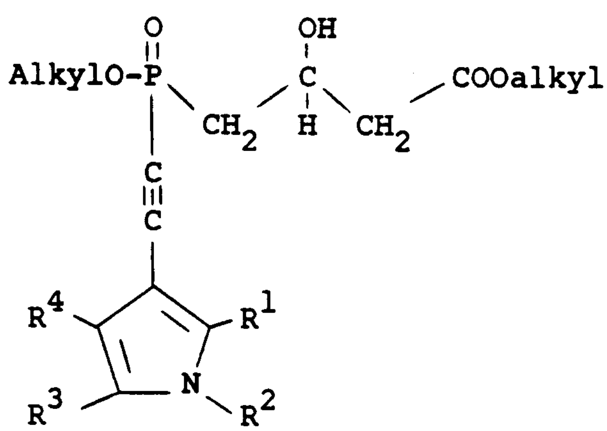

and hydrolysing the above ester with an alkali metal hydroxide to form the corresponding dialkali metal salt.

European Patent Office

# EUROPEAN SEARCH REPORT

Application Number

**EP 91 10 9547**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | FR-A-2 615 516 (SQUIBB)<br>* The whole document<br>* & GB-A-2 205 838 (SQUIBB) (Cat. D.) | 1-15 | C 07 F 9/572<br>A 61 K 31/675<br>C 07 D 207/32 |
| D,Y | FR-A-2 596 393 (SANOFI)<br>* Claims * | 1-10 | |
| Y | EP-A-0 300 278 (BAYER)<br>* Claims * | 1-10 | |
| Y | EP-A-0 287 890 (JOKY-PLAST)<br>* Claims; examples 5,6; tables 1,2 * | 1-10 | |

TECHNICAL FIELDS SEARCHED (Int. Cl.5)

C 07 F 9/00
A 61 K 31/00
C 07 D 207/00

The present search report has been drawn up for all claims

| Place of search | Date of completion of search | Examiner |
|---|---|---|
| The Hague | 19 September 91 | OUSSET J-B. |

CATEGORY OF CITED DOCUMENTS

X : particularly relevant if taken alone
Y : particularly relevant if combined with another document of the same catagory
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document